# EUROPEAN PATENT APPLICATION

(11) **EP 4 596 017 A2**
(43) Date of publication of application: **06.08.2025**
(21) Application number: 25182419.9
(22) Date of filing: 20.07.2018
(51) Int. Cl.: A61M 25/00

(54) **FLEXIBLE ELONGATE DEVICE SYSTEMS AND METHODS**

(30) Priority: 21.07.2017 US 201762535673 P
(62) Divisional of application: 18835711.5
(71) Applicant: Intuitive Surgical Operations, Inc., Sunnyvale, CA 94086-5304 (US)
(72) Inventor: CHAN, Jason K., Sunnyvale, 94086 (US); CALLOL, Joseph R., Sunnyvale, 94086 (US); GORDON, Lucas S., Sunnyvale, 94086 (US); KOWSHIK, Anoop B., Sunnyvale, 94086 (US); SCHLESINGER, Randall L., Sunnyvale, 94086 (US); VALENCIA, Hans F., Sunnyvale, 94086 (US); WALTERS, Worth B., Sunnyvale, 94086 (US)
(74) Representative: J A Kemp LLP

(57) **Abstract**

A steerable catheter comprising: a plurality of control elements; a proximal section including a plurality of conduits to transfer actuation forces applied to one or more of the control elements of the plurality of control elements from a proximal end to a distal end of the proximal section, wherein each control element of the plurality of control elements extends within a respective conduit of the plurality of conduits; a transition section distal to the proximal section, the transition section including a stopper to prevent the plurality of conduits from moving axially along the steerable catheter, wherein the stopper comprises grooves for receiving the one or more control elements; and a distal section distal to the transition section, the transition section including an axial support structure to support the distal section against axial loads generated by the one or more control elements, wherein the stopper prevents the plurality of conduits from extending into the distal section and allows the plurality of control elements to extend into the distal section.

## Description

### RELATED APPLICATION

The present disclosure claims priority to and benefit of U.S. Provisional Patent Application No. 62/535,673, filed July 21, 2017, entitled "Flexible Elongate Device Systems and Methods," which is incorporated by reference herein in its entirety.

### TECHNICAL FIELD

The present disclosure is directed to a support structure for a flexible elongate device.

### BACKGROUND

Minimally invasive medical techniques are intended to reduce the amount of tissue that is damaged during medical procedures, thereby reducing patient recovery time, discomfort, and harmful side effects. Such minimally invasive techniques may be performed through natural orifices in a patient anatomy or through one or more surgical incisions. Through these natural orifices or incisions physician may insert minimally invasive medical instruments (including surgical, diagnostic, therapeutic, or biopsy instruments) to reach a target tissue location. One such minimally invasive technique is to use a flexible and/or steerable elongate device, such as a flexible catheter, that can be inserted into anatomic passageways and navigated toward a region of interest within the patient anatomy. In some applications, the flexible and/or steerable elongate device is subjected to axial loads during operation (e.g., pulling and/or pushing forces along an axial direction of the elongate device). If axial loads exceed the axial strength of the elongate device, the elongate device and/or medical instruments may be damaged and the patient may be injured.

Accordingly, it would be advantageous to provide support structures for flexible and/or steerable elongate devices, such as steerable catheters, that are suitable for use during minimally invasive medical techniques.

### SUMMARY

The embodiments of the invention are best summarized by the claims that follow the description.

According to some embodiments, a flexible elongate device includes a flexible body and an axial support structure. The axial support structure includes at least one groove extending along a length of the axial support structure. The flexible elongate device further includes a plurality of control elements for actuating the flexible elongate device. Each of the plurality of control elements extends through one of the at least one groove of the axial support structure.

According to some embodiments, a method of controlling a flexible elongate device having a flexible body and an axial support structure includes actuating a plurality of control elements, each of the control elements extending through a corresponding one of at least one groove in the axial support structure.

According to some embodiments, a steerable catheter actuated by one or more control elements includes a proximal section including one or more conduits to transfer actuation forces applied to the one or more control elements from distal end to a proximal end of the proximal section, a transition section including a stopper to prevent the one or more conduits from moving axially along the steerable catheter, and a distal section including an axial support structure to support the distal section against axial loads generated by the one or more control elements.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTIONS OF THE DRAWINGS

The patent or application file contains at least one drawing executed in color. Copies of this patent or patent application publication with color drawing(s) will be provided by the Office upon request and payment of the necessary fee.
FIG. 1 is a simplified diagram of a teleoperated medical system according to some embodiments.
FIG. 2A is a simplified diagram of a medical instrument system according to some embodiments.
FIG. 2B is a simplified diagram of a medical instrument with an extended medical tool according to some embodiments.
FIGS. 3A and 3B are simplified diagrams of side views of a patient coordinate space including a medical instrument mounted on an insertion assembly according to some embodiments.
FIGS. 4A-4C are simplified diagrams of a flexible elongate device according to some embodiments.
FIGS. 5A-5C are simplified diagrams of an axial support structure of a flexible elongate device according to some embodiments.
FIGS. 5D and 5E are simplified diagrams of loads and stresses on an axial support structure of a flexible elongate device that is undergoing bending according to some embodiments.
FIG. 5F is a simplified diagram of an alternate axial support hoop structure of a flexible elongate device according to some embodiments.
FIGS. 6A-6B are simplified diagrams of a distal section of a flexible elongate device according to some embodiments.
FIGS. 7A-7B are simplified diagrams of a proximal section of a flexible elongate device according to some embodiments.
FIGS. 8A-8B are simplified diagrams of a stopper of a flexible elongate device according to some embodiments.
FIG. 9 is a simplified diagram of a distal mount of a flexible elongate device according to some embodiments.
FIG. 10 is a simplified diagram of a flexible body with a keyed lumen according to some embodiments.
FIG. 11 is a simplified diagram of an axial support structure of a flexible elongate device according to some embodiments.
FIG. 12 is a simplified diagram of a distal section of a flexible elongate device according to some embodiments.

Embodiments of the present disclosure and their advantages are best understood by referring to the detailed description that follows. It should be appreciated that like reference numerals are used to identify like elements illustrated in one or more of the figures, wherein showings therein are for purposes of illustrating embodiments of the present disclosure and not for purposes of limiting the same.

### DETAILED DESCRIPTION

In the following description, specific details are set forth describing some embodiments consistent with the present disclosure. Numerous specific details are set forth in order to provide a thorough understanding of the embodiments. It will be apparent, however, to one skilled in the art that some embodiments may be practiced without some or all of these specific details. The specific embodiments disclosed herein are meant to be illustrative but not limiting. One skilled in the art may realize other elements that, although not specifically described here, are within the scope and the spirit of this disclosure. In addition, to avoid unnecessary repetition, one or more features shown and described in association with one embodiment may be incorporated into other embodiments unless specifically described otherwise or if the one or more features would make an embodiment non-functional.

In some instances well known methods, procedures, components, and circuits have not been described in detail so as not to unnecessarily obscure aspects of the embodiments.

This disclosure describes various instruments and portions of instruments in terms of their state in three-dimensional space. As used herein, the term "position" refers to the location of an object or a portion of an object in a three-dimensional space (e.g., three degrees of translational freedom along Cartesian x-, y-, and z-coordinates). As used herein, the term "orientation" refers to the rotational placement of an object or a portion of an object (three degrees of rotational freedom - e.g., roll, pitch, and yaw). As used herein, the term "pose" refers to the position of an object or a portion of an object in at least one degree of translational freedom and to the orientation of that object or portion of the object in at least one degree of rotational freedom (up to six total degrees of freedom). As used herein, the term "shape" refers to a set of poses, positions, or orientations measured along an object.

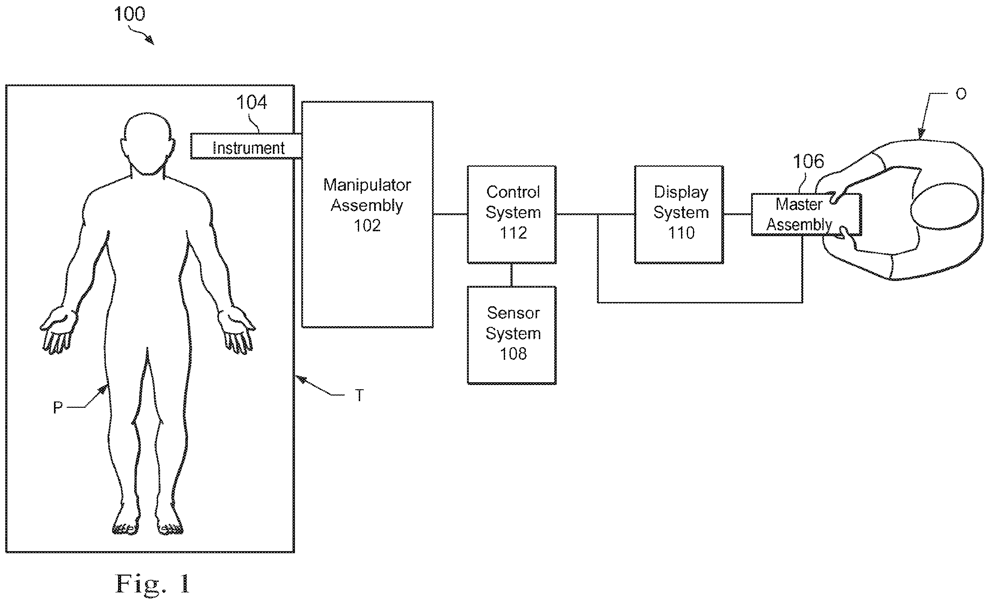

FIG. 1 is a simplified diagram of a teleoperated medical system 100 according to some embodiments. In some embodiments, medical system 100 may be suitable for use in, for example, surgical, diagnostic, therapeutic, or biopsy procedures. While some embodiments are provided herein with respect to such procedures, any reference to medical or surgical instruments and medical or surgical methods is non-limiting. The systems, instruments, and methods described herein may be used for animals, human cadavers, animal cadavers, portions of human or animal anatomy, non-surgical diagnosis, as well as for industrial systems and general robotic, general teleoperational, or robotic medical systems.

As shown in FIG. 1, medical system 100 generally includes a manipulator assembly 102 for operating a medical instrument 104 in performing various procedures on a patient P. Medical instrument 104 may extend into an internal surgical site within the body of patient P via an opening in the body of patient P. The manipulator assembly 102 may be teleoperated, non-teleoperated, or a hybrid teleoperated and non-teleoperated assembly with select degrees of freedom of motion that may be motorized and/or teleoperated and select degrees of freedom of motion that may be non-motorized and/or non-teleoperated. Manipulator assembly 102 is mounted to or near an operating table T. A master assembly 106 allows an operator O (e.g., a surgeon, a clinician, or a physician as illustrated in FIG. 1) to view the interventional site and to control manipulator assembly 102.

Master assembly 106 may be located at a surgeon's console which is usually located in the same room as operating table T, such as at the side of a surgical table on which patient P is located. However, it should be understood that physician O can be located in a different room or a completely different building from patient P. Master assembly 106 generally includes one or more control devices for controlling manipulator assembly 102. The control devices may include any number of a variety of input devices, such as joysticks, trackballs, data gloves, trigger-guns, hand-operated controllers, voice recognition devices, body motion or presence sensors, and/or the like.

Manipulator assembly 102 supports medical instrument 104 and may include a kinematic structure of one or more non-servo controlled links (e.g., one or more links that may be manually positioned and locked in place, generally referred to as a set-up structure), and/or one or more servo controlled links (e.g. one more links that may be controlled in response to commands from the control system), and a manipulator. Manipulator assembly 102 may optionally include a plurality of actuators or motors that drive inputs on medical instrument 104 in response to commands from the control system (e.g., a control system 112). The actuators may optionally include drive systems that when coupled to medical instrument 104 may advance medical instrument 104 into a naturally or surgically created anatomic orifice. Other drive systems may move the distal end of medical instrument 104 in multiple degrees of freedom, which may include three degrees of linear motion (e.g., linear motion along the X, Y, Z Cartesian axes) and in three degrees of rotational motion (e.g., rotation about the X, Y, Z Cartesian axes). Additionally, the actuators can be used to actuate an articulable end effector of medical instrument 104 for grasping tissue in the jaws of a biopsy device and/or the like.

Medical system 100 may include a sensor system 108 with one or more sub-systems for receiving information about the manipulator assembly 102 and/or the medical instrument 104. Such sub-systems may include a position/location sensor system (e.g., an electromagnetic (EM) sensor system); a shape sensor system for determining the position, orientation, speed, velocity, pose, and/or shape of a distal end and/or of one or more segments along a flexible body that may make up medical instrument 104; a visualization system for capturing images from the distal end of medical instrument 104; and actuator position sensors such as resolvers, encoders, potentiometers, and the like that describe the rotation and orientation of the motors controlling the instrument 104.

Medical system 100 also includes a display system 110 for displaying an image or representation of the surgical site and medical instrument 104. Display system 110 and master assembly 106 may be oriented so physician O can control medical instrument 104 and master assembly 106 with the perception of telepresence.

In some embodiments, medical instrument 104 may include a visualization system, which may include an image capture assembly that records a concurrent or real-time image of a surgical site and provides the image to the operator O through one or more displays of display system 110. The concurrent image may be, for example, a two or three dimensional image captured by an endoscope positioned within the surgical site. In some embodiments, the visualization system includes endoscopic components that may be integrally or removably coupled to medical instrument 104. However in some embodiments, a separate endoscope, attached to a separate manipulator assembly may be used with medical instrument 104 to image the surgical site. The visualization system may be implemented as hardware, firmware, software or a combination thereof which interact with or are otherwise executed by one or more computer processors, which may include the processors of a control system 112.

Display system 110 may also display an image of the surgical site and medical instruments captured by the visualization system. In some examples, medical system 100 may configure medical instrument 104 and controls of master assembly 106 such that the relative positions of the medical instruments are similar to the relative positions of the eyes and hands of operator O. In this manner operator O can manipulate medical instrument 104 and the hand control as if viewing the workspace in substantially true presence. By true presence, it is meant that the presentation of an image is a true perspective image simulating the viewpoint of a physician that is physically manipulating medical instrument 104.

In some examples, display system 110 may present images of a surgical site recorded pre-operatively or intra-operatively using image data from imaging technology such as, computed tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. The pre-operative or intra-operative image data may be presented as two-dimensional, three-dimensional, or four-dimensional (including e.g., time based or velocity based information) images and/or as images from models created from the pre-operative or intra-operative image data sets.

In some embodiments, often for purposes of imaged guided medical procedures, display system 110 may display a virtual navigational image in which the actual location of medical instrument 104 is registered (i.e., dynamically referenced) with the preoperative or concurrent images/model. This may be done to present the physician O with a virtual image of the internal surgical site from a viewpoint of medical instrument 104.

Medical system 100 may also include control system 112. Control system 112 includes at least one memory and at least one computer processor (not shown) for effecting control between medical instrument 104, master assembly 106, sensor system 108, and display system 110. Control system 112 also includes programmed instructions (e.g., a non-transitory machine-readable medium storing the instructions) to implement some or all of the methods described in accordance with aspects disclosed herein, including instructions for providing information to display system 110. While control system 112 is shown as a single block in the simplified schematic of FIG. 1, the system may include two or more data processing circuits with one portion of the processing optionally being performed on or adjacent to manipulator assembly 102, another portion of the processing being performed at master assembly 106, and/or the like. The processors of control system 112 may execute instructions comprising instruction corresponding to processes disclosed herein and described in more detail below.

In some embodiments, control system 112 may receive force and/or torque feedback from medical instrument 104. Responsive to the feedback, control system 112 may transmit signals to master assembly 106. In some examples, control system 112 may transmit signals instructing one or more actuators of manipulator assembly 102 to move medical instrument 104.

Control system 112 may optionally further include a virtual visualization system to provide navigation assistance to operator O when controlling medical instrument 104 during an image-guided medical procedure. Virtual navigation using the virtual visualization system may be based upon reference to an acquired preoperative or intraoperative dataset of anatomic passageways. Software, which may be used in combination with operator inputs, is used to convert the recorded images into segmented two dimensional or three dimensional composite representation of a partial or an entire anatomic organ or anatomic region. An image data set is associated with the composite representation. The virtual visualization system obtains sensor data from sensor system 108 that is used to compute an approximate location of medical instrument 104 with respect to the anatomy of patient P. The system may implement the sensor system 108 to register and display the medical instrument together with the preoperatively or intraoperatively recorded surgical images. For example, PCT Publication WO 2016/191298 (published December 1, 2016) (disclosing "Systems and Methods of Registration for Image Guided Surgery"), which is incorporated by reference herein in its entirety, discloses such one system.

During a virtual navigation procedure, sensor system 108 may be used to compute an approximate location of medical instrument 104 with respect to the anatomy of patient P. The location can be used to produce both macro-level (external) tracking images of the anatomy of patient P and virtual internal images of the anatomy of patient P. The system may implement one or more electromagnetic (EM) sensor, fiber optic sensors, and/or other sensors to register and display a medical implement together with preoperatively recorded surgical images. For example U.S. Patent No. 8,900,131 (filed May 13, 2011) (disclosing "Medical System Providing Dynamic Registration of a Model of an Anatomic Structure for Image-Guided Surgery") which is incorporated by reference herein in its entirety, discloses one such system.

Medical system 100 may further include optional operations and support systems (not shown) such as illumination systems, steering control systems, irrigation systems, and/or suction systems. In some embodiments, medical system 100 may include more than one manipulator assembly and/or more than one master assembly. The exact number of teleoperational manipulator assemblies will depend on the medical procedure and the space constraints within the operating room, among other factors. Master assembly 106 may be collocated or they may be positioned in separate locations. Multiple master assemblies allow more than one operator to control one or more teleoperational manipulator assemblies in various combinations.

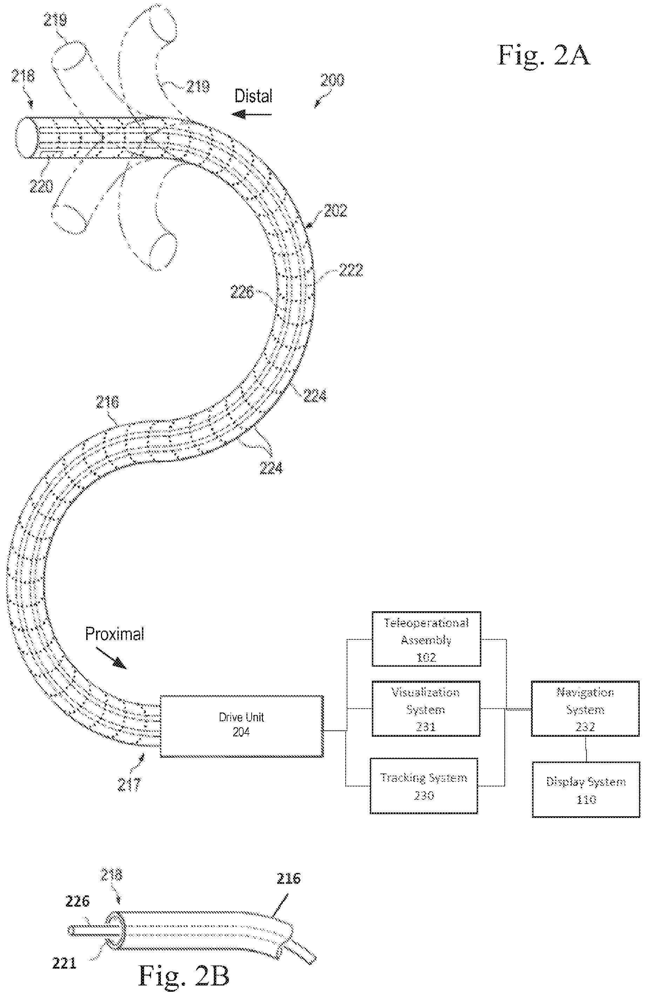

FIG. 2A is a simplified diagram of a medical instrument system 200 according to some embodiments. In some embodiments, medical instrument system 200 may be used as medical instrument 104 in an image-guided medical procedure performed with teleoperated medical system 100. In some examples, medical instrument system 200 may be used for non-teleoperational exploratory procedures or in procedures involving traditional manually operated medical instruments, such as endoscopy. Optionally medical instrument system 200 may be used to gather (i.e., measure) a set of data points corresponding to locations within anatomic passageways of a patient, such as patient P.

Medical instrument system 200 includes elongate device 202 coupled to a drive unit 204. Elongate device 202 includes a flexible body 216 having proximal end 217 and distal end or tip portion 218. In some embodiments, flexible body 216 has an approximately 3 mm outer diameter. Other flexible body outer diameters may be larger or smaller.

Medical instrument system 200 further includes a tracking system 230 for determining the position, orientation, speed, velocity, pose, and/or shape of flexible body 216 at distal end 218 and/or of one or more segments 224 along flexible body 216 using one or more sensors and/or imaging devices as described in further detail below. The entire length of flexible body 216, between distal end 218 and proximal end 217, may be effectively divided into segments 224. If medical instrument system 200 is consistent with medical instrument 104 of a medical system 100, tracking system 230. Tracking system 230 may optionally be implemented as hardware, firmware, software or a combination thereof which interact with or are otherwise executed by one or more computer processors, which may include the processors of control system 112 in FIG. 1.

Tracking system 230 may optionally track distal end 218 and/or one or more of the segments 224 using a shape sensor 222. Shape sensor 222 may optionally include an optical fiber aligned with flexible body 216 (e.g., provided within an interior channel (not shown) or mounted externally). In one embodiment, the optical fiber has a diameter of approximately 200 µm. In other embodiments, the dimensions may be larger or smaller. The optical fiber of shape sensor 222 forms a fiber optic bend sensor for determining the shape of flexible body 216. In one alternative, optical fibers including Fiber Bragg Gratings (FBGs) are used to provide strain measurements in structures in one or more dimensions. Various systems and methods for monitoring the shape and relative position of an optical fiber in three dimensions are described in U.S. Patent Application Publication No. 2006/0013523 (filed July 13, 2005) (disclosing "Fiber optic position and shape sensing device and method relating thereto"); U.S. Patent No. 7,772,541 (filed on Jul. 16, 2004) (disclosing "Fiber-optic shape and relative position sensing"); and U.S. Patent No. 6,389,187 (filed on Jun. 17, 1998) (disclosing "Optical Fibre Bend Sensor"), which are all incorporated by reference herein in their entireties. Sensors in some embodiments may employ other suitable strain sensing techniques, such as Rayleigh scattering, Raman scattering, Brillouin scattering, and Fluorescence scattering. In some embodiments, the shape of flexible body 216 may be determined using other techniques. For example, a history of the distal end pose of flexible body 216 can be used to reconstruct the shape of flexible body 216 over the interval of time. In some embodiments, tracking system 230 may optionally and/or additionally track distal end 218 using a position sensor system 220. Position sensor system 220 may be a component of an EM sensor system with positional sensor system 220 including one or more conductive coils that may be subjected to an externally generated electromagnetic field. Each coil of EM sensor system 220 then produces an induced electrical signal having characteristics that depend on the position and orientation of the coil relative to the externally generated electromagnetic field. In some embodiments, position sensor system 220 may be configured and positioned to measure six degrees of freedom, e.g., three position coordinates X, Y, Z and three orientation angles indicating pitch, yaw, and roll of a base point or five degrees of freedom, e.g., three position coordinates X, Y, Z and two orientation angles indicating pitch and yaw of a base point. Further description of a position sensor system is provided in U.S. Patent No. 6,380,732 (filed August 11, 1999) (disclosing "Six-Degree of Freedom Tracking System Having a Passive Transponder on the Object Being Tracked"), which is incorporated by reference herein in its entirety.

In some embodiments, tracking system 230 may alternately and/or additionally rely on historical pose, position, or orientation data stored for a known point of an instrument system along a cycle of alternating motion, such as breathing. This stored data may be used to develop shape information about flexible body 216. In some examples, a series of positional sensors (not shown), such as electromagnetic (EM) sensors similar to the sensors in position sensor 220 may be positioned along flexible body 216 and then used for shape sensing. In some examples, a history of data from one or more of these sensors taken during a procedure may be used to represent the shape of elongate device 202, particularly if an anatomic passageway is generally static.

Flexible body 216 includes a channel 221 sized and shaped to receive a medical instrument 226. FIG. 2B is a simplified diagram of flexible body 216 with medical instrument 226 extended according to some embodiments. In some embodiments, medical instrument 226 may be used for procedures such as surgery, biopsy, ablation, illumination, irrigation, or suction. Medical instrument 226 can be deployed through channel 221 of flexible body 216 and used at a target location within the anatomy. Medical instrument 226 may include, for example, image capture probes, biopsy instruments, laser ablation fibers, and/or other surgical, diagnostic, or therapeutic tools. Medical tools may include end effectors having a single working member such as a scalpel, a blunt blade, an optical fiber, an electrode, and/or the like. Other end effectors may include, for example, forceps, graspers, scissors, clip appliers, and/or the like. Other end effectors may further include electrically activated end effectors such as electrosurgical electrodes, transducers, sensors, and/or the like. In various embodiments, medical instrument 226 is a biopsy instrument, which may be used to remove sample tissue or a sampling of cells from a target anatomic location. Medical instrument 226 may be used with an image capture probe also within flexible body 216. In various embodiments, medical instrument 226 may be an image capture probe that includes a distal portion with a stereoscopic or monoscopic camera at or near distal end 218 of flexible body 216 for capturing images (including video images) that are processed by a visualization system 231 for display and/or provided to tracking system 230 to support tracking of distal end 218 and/or one or more of the segments 224. The image capture probe may include a cable coupled to the camera for transmitting the captured image data. In some examples, the image capture instrument may be a fiber-optic bundle, such as a fiberscope, that couples to visualization system 231. The image capture instrument may be single or multispectral, for example capturing image data in one or more of the visible, infrared, and/or ultraviolet spectrums. Alternatively, medical instrument 226 may itself be the image capture probe. Medical instrument 226 may be advanced from the opening of channel 221 to perform the procedure and then retracted back into the channel when the procedure is complete. Medical instrument 226 may be removed from proximal end 217 of flexible body 216 or from another optional instrument port (not shown) along flexible body 216.

Medical instrument 226 may additionally house cables, linkages, or other actuation controls (not shown) that extend between its proximal and distal ends to controllably the bend distal end of medical instrument 226. Steerable instruments are described in detail in U.S. Patent No. 7,316,681 (filed on Oct. 4, 2005) (disclosing "Articulated Surgical Instrument for Performing Minimally Invasive Surgery with Enhanced Dexterity and Sensitivity") and U.S. Patent No. 9,259,274 (filed Sept. 30, 2008) (disclosing "Passive Preload and Capstan Drive for Surgical Instruments"), which are incorporated by reference herein in their entireties.

Flexible body 216 may also house cables, linkages, or other steering controls (not shown) that extend between drive unit 204 and distal end 218 to controllably bend distal end 218 as shown, for example, by broken dashed line depictions 219 of distal end 218. In some examples, at least four cables are used to provide independent "up-down" steering to control a pitch of distal end 218 and "left-right" steering to control a yaw of distal end 281. Steerable catheters are described in detail in U.S. Patent No. 9,452,276 (filed Oct. 14, 2011) (disclosing "Catheter with Removable Vision Probe"), which is incorporated by reference herein in its entirety. In embodiments in which medical instrument system 200 is actuated by a teleoperational assembly, drive unit 204 may include drive inputs that removably couple to and receive power from drive elements, such as actuators, of the teleoperational assembly. In some embodiments, medical instrument system 200 may include gripping features, manual actuators, or other components for manually controlling the motion of medical instrument system 200. Elongate device 202 may be steerable or, alternatively, the system may be non-steerable with no integrated mechanism for operator control of the bending of distal end 218. In some examples, one or more lumens, through which medical instruments can be deployed and used at a target surgical location, are defined in the walls of flexible body 216.

In some embodiments, medical instrument system 200 may include a flexible bronchial instrument, such as a bronchoscope or bronchial catheter, for use in examination, diagnosis, biopsy, or treatment of a lung. Medical instrument system 200 is also suited for navigation and treatment of other tissues, via natural or surgically created connected passageways, in any of a variety of anatomic systems, including the colon, the intestines, the kidneys and kidney calices, the brain, the heart, the circulatory system including vasculature, and/or the like.

The information from tracking system 230 may be sent to a navigation system 232 where it is combined with information from visualization system 231 and/or the preoperatively obtained models to provide the physician, clinician, or surgeon or other operator with real-time position information. In some examples, the real-time position information may be displayed on display system 110 of FIG. 1 for use in the control of medical instrument system 200. In some examples, control system 116 of FIG. 1 may utilize the position information as feedback for positioning medical instrument system 200. Various systems for using fiber optic sensors to register and display a surgical instrument with surgical images are provided in U.S. Patent No. 8,900,131, filed May 13, 2011, disclosing, "Medical System Providing Dynamic Registration of a Model of an Anatomic Structure for Image-Guided Surgery," which is incorporated by reference herein in its entirety.

In some examples, medical instrument system 200 may be teleoperated within medical system 100 of FIG. 1. In some embodiments, manipulator assembly 102 of FIG. 1 may be replaced by direct operator control. In some examples, the direct operator control may include various handles and operator interfaces for hand-held operation of the instrument.

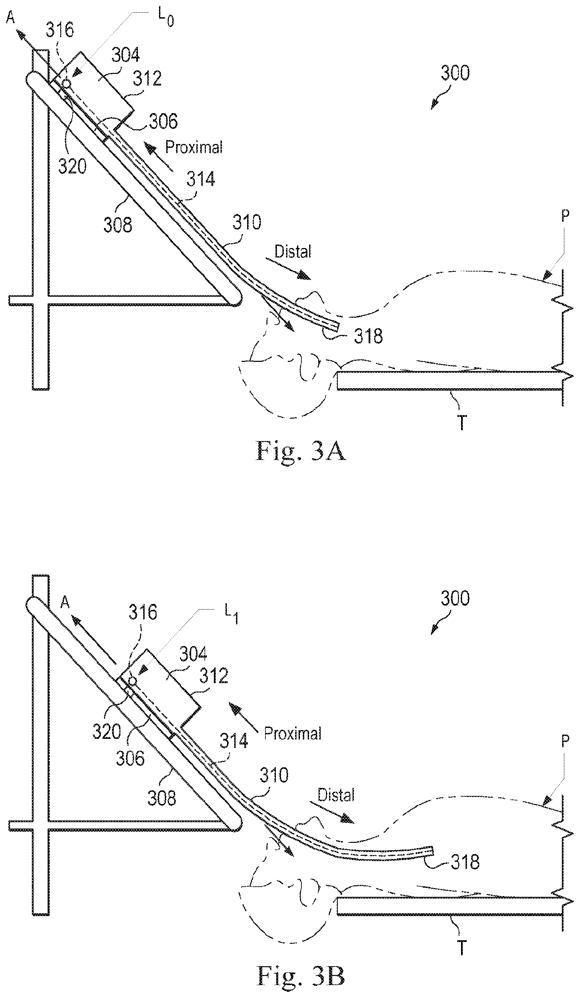

FIGS. 3A and 3B are simplified diagrams of side views of a patient coordinate space including a medical instrument mounted on an insertion assembly according to some embodiments. As shown in FIGS. 3A and 3B, a surgical environment 300 including a patient P is positioned on the table T of FIG. 1. Patient P may be stationary within the surgical environment in the sense that gross patient movement is limited by sedation, restraint, and/or other means. Cyclic anatomic motion including respiration and cardiac motion of patient P may continue. Within surgical environment 300, a medical instrument 304 is used to perform a medical procedure which may include, for example, surgery, biopsy, ablation, illumination, irrigation, suction, or a system registration procedure. The medical instrument 304 may be, for example, the instrument 104. The instrument 304 includes a flexible elongate device 310 (e.g., a catheter) coupled to an instrument body 312. Elongate device 310 includes one or more channels (not shown) sized and shaped to receive a medical tool (not shown).

Elongate device 310 may also include one or more sensors (e.g., components of the sensor system 108). In some embodiments, an optical fiber shape sensor 314 is fixed at a proximal point 316 on instrument body 312. In some embodiments, proximal point 316 of optical fiber shape sensor 314 may be movable along with instrument body 312 but the location of proximal point 316 may be known (e.g., via a tracking sensor or other tracking device). Shape sensor 314 measures a shape from proximal point 316 to another point such as distal end 318 of elongate device 310. Shape sensor 314 may be aligned with flexible elongate device 310 (e.g., provided within an interior channel (not shown) or mounted externally). In one embodiment, the optical fiber has a diameter of approximately 200 µm. In other embodiments, the dimensions may be larger or smaller. The shape sensor 314 may be used to determine the shape of flexible elongate device 310. In one alternative, optical fibers including Fiber Bragg Gratings (FBGs) are used to provide strain measurements in structures in one or more dimensions. Various systems and methods for monitoring the shape and relative position of an optical fiber in three dimensions are described in U.S. Patent Application Publication No. 2006/0013523 (filed July 13, 2005) (disclosing "Fiber optic position and shape sensing device and method relating thereto"); U.S. Patent No. 7,772,541 (filed on Jul. 16, 2004) (disclosing "Fiber-optic shape and relative position sensing"); and U.S. Patent No. 6,389,187 (filed on Jun. 17, 1998) (disclosing "Optical Fibre Bend Sensor"), which are all incorporated by reference herein in their entireties. Sensors in some embodiments may employ other suitable strain sensing techniques, such as Rayleigh scattering, Raman scattering, Brillouin scattering, and Fluorescence scattering. Various systems for using fiber optic sensors to register and display a surgical instrument with surgical images are provided in PCT Publication WO 2016/191298 (published December 1, 2016) (disclosing "Systems and Methods of Registration for Image Guided Surgery"), which is incorporated by reference herein in its entirety.

In various embodiments, position sensors such as electromagnetic (EM) sensors, may be incorporated into the medical instrument 304. In various embodiments, a series of position sensors may be positioned along the flexible elongate device 310 and then used for shape sensing. In some embodiments, position sensors may be configured and positioned to measure six degrees of freedom, e.g., three position coordinates X, Y, Z and three orientation angles indicating pitch, yaw, and roll of a base point or five degrees of freedom, e.g., three position coordinates X, Y, Z and two orientation angles indicating pitch and yaw of a base point. Further description of a position sensor system is provided in U.S. Patent No. 6,380,732 (filed August 11, 1999) (disclosing "Six-Degree of Freedom Tracking System Having a Passive Transponder on the Object Being Tracked"), which is incorporated by reference herein in its entirety.

Elongate device 310 may also house cables, linkages, or other steering controls (not shown) that extend between instrument body 312 and distal end 318 to controllably bend distal end 318. In some examples, at least four cables are used to provide independent "up-down" steering to control a pitch of distal end 318 and "left-right" steering to control a yaw of distal end 318. Steerable elongate devices are described in detail in U.S. Patent No. 9,452,276 (filed Oct. 14, 2011) (disclosing "Catheter with Removable Vision Probe"), which is incorporated by reference herein in its entirety. The instrument body 312 may include drive inputs that removably couple to and receive power from drive elements, such as actuators, of the teleoperational assembly.

A patient P is positioned on the table T of FIG. 1. Patient P may be stationary within the surgical environment in the sense that gross patient movement is limited by sedation, restraint, and/or other means. Cyclic anatomic motion including respiration and cardiac motion of patient P may continue. Within surgical environment 300, a medical instrument 304 is used to perform a medical procedure which may include, for example, surgery, biopsy, ablation, illumination, irrigation, suction, or a system registration procedure. The medical instrument 304 may be, for example, the instrument 104. The instrument 304 includes a flexible elongate device 310 (e.g., a catheter) coupled to an instrument body 312. Elongate device 310 includes one or more channels (not shown) sized and shaped to receive a medical tool (not shown).

Elongate device 310 may also include one or more sensors (e.g., components of the sensor system 108). In some embodiments, an optical fiber shape sensor 314 is fixed at a proximal point 316 on instrument body 312. In some embodiments, proximal point 316 of optical fiber shape sensor 314 may be movable along with instrument body 312 but the location of proximal point 316 may be known (e.g., via a tracking sensor or other tracking device). Shape sensor 314 measures a shape from proximal point 316 to another point such as distal end 318 of elongate device 310. Shape sensor 314 may be aligned with flexible elongate device 310 (e.g., provided within an interior channel (not shown) or mounted externally). In one embodiment, the optical fiber has a diameter of approximately 200 µm. In other embodiments, the dimensions may be larger or smaller. The shape sensor 314 may be used to determine the shape of flexible elongate device 310. In one alternative, optical fibers including Fiber Bragg Gratings (FBGs) are used to provide strain measurements in structures in one or more dimensions. Various systems and methods for monitoring the shape and relative position of an optical fiber in three dimensions are described in U.S. Patent Application Publication No. 2006/0013523 (filed July 13, 2005) (disclosing "Fiber optic position and shape sensing device and method relating thereto"); U.S. Patent No. 7,772,541 (filed on Jul. 16, 2004) (disclosing "Fiber-optic shape and relative position sensing"); and U.S. Patent No. 6,389,187 (filed on Jun. 17, 1998) (disclosing "Optical Fibre Bend Sensor"), which are all incorporated by reference herein in their entireties. Sensors in some embodiments may employ other suitable strain sensing techniques, such as Rayleigh scattering, Raman scattering, Brillouin scattering, and Fluorescence scattering. Various systems for using fiber optic sensors to register and display a surgical instrument with surgical images are provided in PCT Publication WO 2016/191298 (published December 1, 2016) (disclosing "Systems and Methods of Registration for Image Guided Surgery"), which is incorporated by reference herein in its entirety.

In various embodiments, position sensors such as electromagnetic (EM) sensors, may be incorporated into the medical instrument 304. In various embodiments, a series of position sensors may be positioned along the flexible elongate device 310 and then used for shape sensing. In some embodiments, position sensors may be configured and positioned to measure six degrees of freedom, e.g., three position coordinates X, Y, Z and three orientation angles indicating pitch, yaw, and roll of a base point or five degrees of freedom, e.g., three position coordinates X, Y, Z and two orientation angles indicating pitch and yaw of a base point. Further description of a position sensor system is provided in U.S. Patent No. 6,380,732 (filed August 11, 1999) (disclosing "Six-Degree of Freedom Tracking System Having a Passive Transponder on the Object Being Tracked"), which is incorporated by reference herein in its entirety.

Elongate device 310 may also house cables, linkages, or other steering controls (not shown) that extend between instrument body 312 and distal end 318 to controllably bend distal end 318. In some examples, at least four cables are used to provide independent "up-down" steering to control a pitch of distal end 318 and "left-right" steering to control a yaw of distal end 318. Steerable elongate devices are described in detail in U.S. Patent No. 9,452,276 (filed Oct. 14, 2011) (disclosing "Catheter with Removable Vision Probe"), which is incorporated by reference herein in its entirety. The instrument body 312 may include drive inputs that removably couple to and receive power from drive elements, such as actuators, of the teleoperational assembly.

Instrument body 312 may be coupled to instrument carriage 306. Instrument carriage 306 is mounted to an insertion stage 308 fixed within surgical environment 300. Alternatively, insertion stage 308 may be movable but have a known location (e.g., via a tracking sensor or other tracking device) within surgical environment 300. Instrument carriage 306 may be a component of a manipulator assembly (e.g., manipulator assembly 102) that couples to medical instrument 304 to control insertion motion (i.e., motion along the A axis) and, optionally, motion of a distal end 318 of an elongate device 310 in multiple directions including yaw, pitch, and roll. Instrument carriage 306 or insertion stage 308 may include actuators, such as servomotors, (not shown) that control motion of instrument carriage 306 along insertion stage 308.

A sensor device 320, which may be a component of the sensor system 108, provides information about the position of instrument body 312 as it moves on insertion stage 308 along an insertion axis A. Sensor device 320 may include resolvers, encoders, potentiometers, and/or other sensors that determine the rotation and/or orientation of the actuators controlling the motion of instrument carriage 306 and consequently the motion of instrument body 312. In some embodiments, insertion stage 308 is linear. In some embodiments, insertion stage 308 may be curved or have a combination of curved and linear sections.

FIG. 3A shows instrument body 312 and instrument carriage 306 in a retracted position along insertion stage 308. In this retracted position, the proximal point 316 is at a position L0 on axis A. In this position along insertion stage 308, the location of proximal point 316 may be set to a zero and/or another reference value to provide a base reference to describe the position of instrument carriage 306, and thus proximal point 316, on insertion stage 308. With this retracted position of instrument body 312 and instrument carriage 306, distal end 318 of elongate device 310 may be positioned just inside an entry orifice of patient P. Also in this position, sensor device 320 may be set to a zero and/or another reference value (e.g., I=0). In FIG. 3B, instrument body 312 and instrument carriage 306 have advanced along the linear track of insertion stage 308 and distal end 318 of elongate device 310 has advanced into patient P. In this advanced position, the proximal point 316 is at a position L1 on the axis A. In some examples, encoder and/or other position data from one or more actuators controlling movement of instrument carriage 306 along insertion stage 308 and/or one or more position sensors associated with instrument carriage 306 and/or insertion stage 308 is used to determine the position Lx of proximal point 316 relative to position L0. In some examples, position Lx may further be used as an indicator of the distance or insertion depth to which distal end 318 of elongate device 310 is inserted into the passageways of the anatomy of patient P.

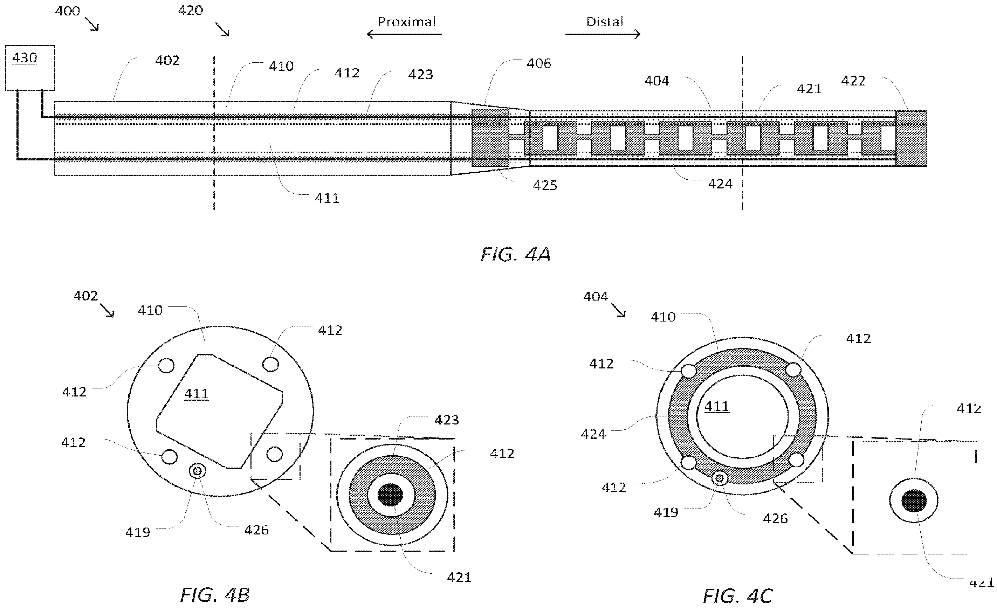

FIGS. 4A-4C are simplified diagrams of a flexible elongate device 400 according to some embodiments. According to some embodiments consistent with FIGS. 1-3, flexible elongate device 400 may correspond to elongate device 202 of medical instrument system 200. As depicted in FIG. 4A, flexible elongate device 400 can include a proximal section 402, a distal section 404 and a transition section 406 therebetween.

Flexible elongate device 400 can include a flexible body 410 with a flexible wall having a thickness extending from an inner surface to an outer surface of the flexible body 410. A main lumen 411 can extend within the flexible body 410, through the proximal section 402, the transition section 406, and the distal section 404. The main lumen 411 can provide a delivery channel for a medical tool, such as an endoscope, biopsy needle, endobronchial ultrasound (EBUS) probe, ablation tool, chemical delivery tool, and/or the like, to be inserted through flexible body 410. According to some embodiments, a plurality of control element lumens 412 extend through the flexible wall of the flexible body 410 arranged circumferentially in the flexible wall around the main lumen 411. According to some embodiments, a sensor lumen 419 extends through the flexible wall of the flexible body 410. The sensor lumen 419 can extend from the proximal end of the flexible elongate device 400, through the proximal section 402, transition section 406, terminating at a distal portion of the distal section 404. In some examples, flexible body 410 may include various other types of lumens for electrical wires, fibers, sensors, small medical instruments, chemical delivery, and/or the like. In alternative embodiments, flexible body 410 may include an all-purpose lumen that can be used for a variety of purposes, including accommodating multiple concurrently inserted instruments, control elements, sensors, and/or the like.

As depicted in FIGS. 4A-4C, within each of the control element lumens 412, a coil pipe or conduit 423 can extend through the proximal section 402 of the flexible body 410, providing channels through which a plurality of control elements 421 extends. In some examples, control elements 421 can include pull wires, tendons, push rods and/or the like. The conduits 423 terminate at the transition section 406, proximal to the distal section 404. The control elements 421 extend out of the conduits 423 at the transition section 406, entering the distal section 404 through control element lumens 412, and attach to a distal mount 422. The one or more control elements 421 can be used to actuate distal section 404 of flexible elongate device 400. As depicted in FIGS. 4B and 4C, four control elements 421 can be disposed within control element lumens 412 and evenly spaced around the circumference of flexible elongate device 400.

In the illustrative example provided in FIG. 4B, a pair of lumens that includes sensor lumen 419 and one of control element lumens 412 is approximately centered along a side of the rounded square formed by main lumen 411. Consequently, neither sensor lumen 419 nor control element lumens 412 are individually centered along the side of the rounded square. Moreover, because control elements 421 are equally spaced around the perimeter of the rounded square, none of the control element lumens 412 are centered relative to the rounded square. As a result, the square formed by control elements 421 and the rounded square formed by main lumen 411 are offset by approximately 30 degrees. However, it is to be understood that different numbers and/or arrangements of control elements 421 are possible. For example, control elements 421 may be unevenly spaced around the circumference of flexible elongate device 400 and/or centered relative to the sides of main lumen 411.

Distal section 404 is actuated by applying actuation forces to control elements 421 (e.g., pulling and/or pushing on control elements 421 in an unequal manner). Applying actuation forces causes distal section 404 to bend in the direction defined by the net actuation forces. In some examples, the actuation forces may be applied manually, robotically, and/or the like. For example, the actuation forces may be applied using an actuator 430 positioned at the proximal end of flexible elongate device 400. Conduits 423 transfer the actuation forces applied to control elements 421 from the proximal end to the distal end of proximal section 402 at transition section 406. Consequently, even when unequal actuation forces are applied to control elements 421, little actuation force appears within proximal section 402. In some examples, conduits 423 may be flexible to retain the flexibility of proximal section 402. Further examples of conduits are provided in P.C.T. Patent Application PCT/US14/62188 entitled "Flexible Instrument with Embedded Actuation Conduits," filed October 24, 2014, which is hereby incorporated by reference in its entirety.

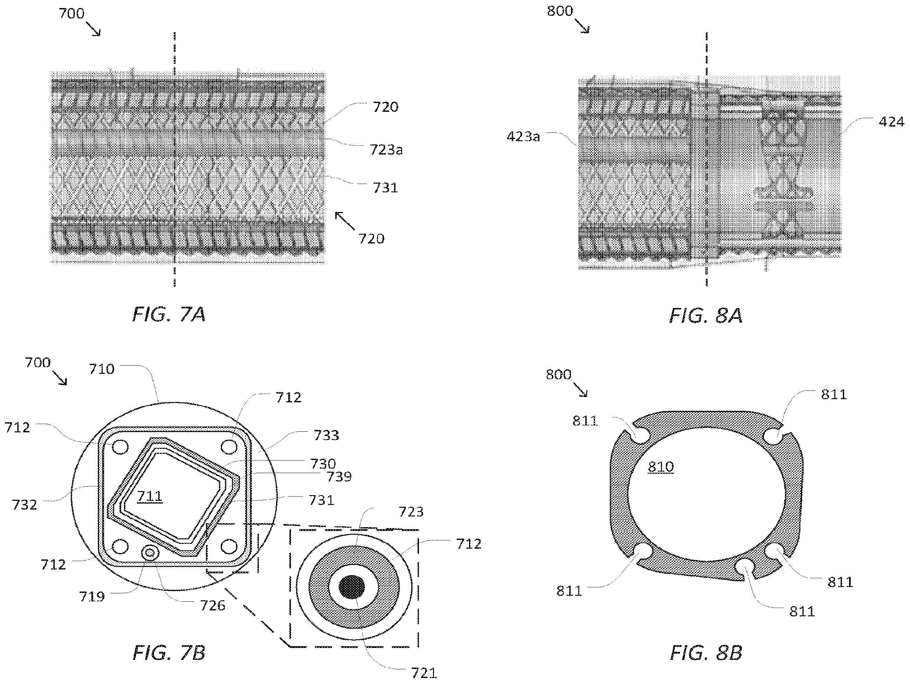

Any bend along the length of the proximal section 402 of the flexible elongate device 400 results in a change of length of control element lumens 412. For example with reference to FIG. 4A, if the flexible body bends in a downward motion, the control element lumens 412 on the lower portion of flexible body 410 will decrease in length while the control element lumens 412 on the upper portion of flexible body will increase in length. Thus it can be necessary for the conduits 423 to axially slide within the control element lumens 412. In some examples, the conduits can be constrained (e.g., fixed and/or prevented from sliding proximally along a conduit longitudinal axis) at a proximal end of the flexible elongate device within the actuator 430 and can terminate at the transition section 406. The conduits may be constrained (e.g., fixed and/or prevented from sliding distally along a conduit longitudinal axis) at the transition section 406. In this example, within transition section 406, a stopper 425 is coupled between conduits 423 on the stopper proximal side and an axial support structure 424 on the distal stopper side. Stopper 425 prevents conduits 423 from shifting distally along flexible elongate device 400. In alternative examples, the conduits may be fixed to stopper 425. Examples of stoppers are discussed in greater detail below with reference to FIGS. 8A-8B.

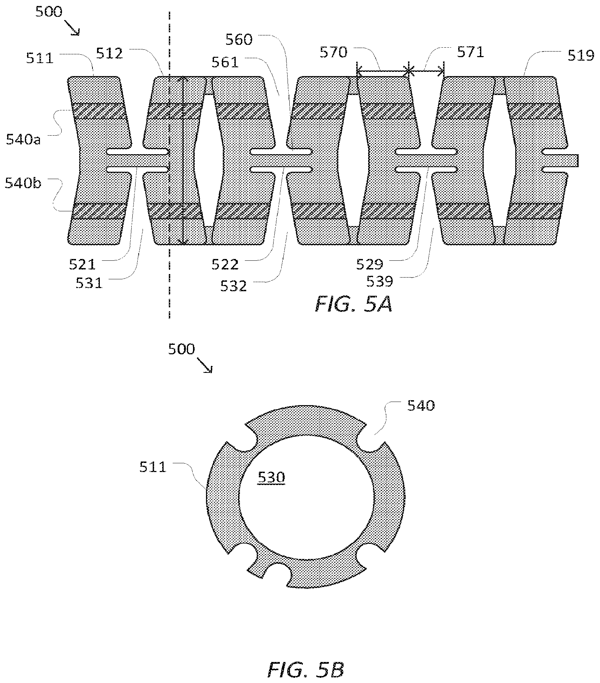

Within distal section 404, axial support structure 424 is configured to bend in response to actuation forces applied to control elements 421. Consequently, when unequal actuation forces are applied to control elements 421, distal section 404 bends in the direction defined by the net actuation forces. Axial support structure 424 supports distal section 404 against axial loads generated by the actuation forces applied to control elements 421. In particular, axial support structure 424 may prevent or reduce distortion, compression and/or collapse of distal section 404 under axial loads. Examples of axial support structures are discussed in greater detail below with reference to FIGS. 5A-5C. Further examples of axial support structures are provided in U.S. Provisional Patent Application 62/378,943 entitled "Axial Support Structure for a Flexible Elongate Device," which is hereby incorporated by reference in its entirety.

Although axial support structure 424 is depicted as having a spine-like structure in FIG. 4A, other structures are possible. For example, axial support structure 424 may be composed of conduits similar to conduits 423. Whereas the conduits of conduits 423 are arranged concentrically around control elements 421 to counteract the actuation forces applied to control elements 421, the conduits of axial support structure 424 may be offset from control elements 421 (e.g., located at different positions around the circumference of flexible body 410) to allow axial support structure 424 to bend in response to actuation forces. Alternately or additionally, the conduits of axial support structure 424 may be more flexible (e.g., smaller diameter and/or constructed using smaller gauge wire) than conduits 423. In some examples, axial support structure 424 may be formed as a single large coil that encloses main lumen 411.

In some examples, one or more of lumens 411, 412, 419, and/or sections thereof, may be keyed. That is, a lumen and/or a portion of a lumen may have a non-circular cross-sectional shape that prevents or constrains the rotation of a tool (e.g., a medical instrument, sensor, fiber, electrical wire, actuation element, and/or the like) with a matching non-circular cross-sectional shape when inserted through the lumen. As depicted in FIG. 4B, main lumen 411 of proximal section 402 is keyed. In particular, main lumen 411 has a rounded square cross-sectional shape that supports four keyed orientations.

In some examples, a localization sensor 426, such as an optical fiber of shape sensor 222, extends through sensor lumen 419. Like conduits 423, localization sensor may be constrained (e.g., fixedly attached and/or prevented from sliding axially) at each end of flexible elongate device 420. In one example, the localization sensor is fixedly attached to distal mount 422, free-floating within sensor lumen 419, and fixedly attached at actuator 430. In some examples, a service loop can be provided within actuator 430 between a fixed localization attachment and a proximal end of flexible elongate device 420 to accommodate the varying length of sensor lumen 419 due to bending. In alternative examples, the service loop can be provided between actuator 430 and the distal end of flexible elongate device 420 or within the flexible elongate device.

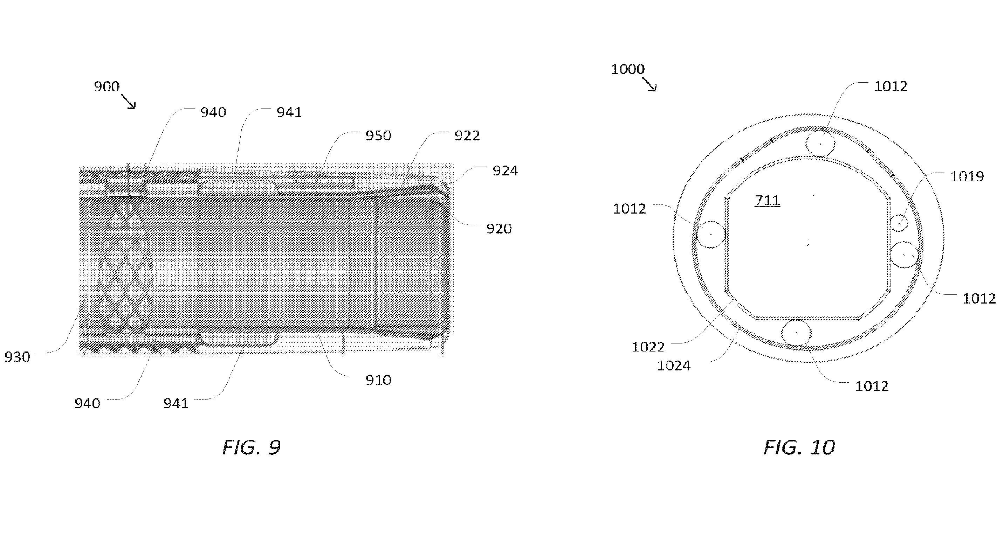

In some examples, the cross sectional shape of lumens 411-419 may change between proximal section 402 and distal section 404. For example, main lumen 411 may be keyed within proximal section 402 and unkeyed within distal section 404. As depicted in FIG. 4C, main lumen 411 of distal section 404 is unkeyed, having a circular cross-sectional shape that does not constrain the rotation of a medical instrument inserted therein. Examples of keyed lumens are discussed in greater detail below with reference to FIG. 10.

In some examples, the diameter of lumens 411-419 may change between proximal section 402 and distal section 404. Accordingly, lumens 411-419 may be tapered within transition section 406 to provide a gradual transition between the different cross-sectional shapes, e.g., a keyed lumen on the proximal side and an unkeyed lumen on the distal side.

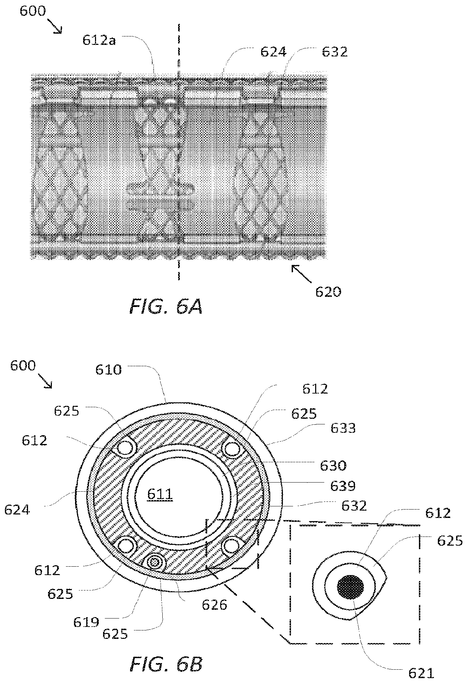

In some examples, the flexible wall of the flexible body 410 may vary between the proximal section 402 and distal section 404. In some examples, a required bending flexibility and/or compressive strength may vary along the length of the catheter based on potential positioning within patient anatomy. Thus the flexible wall may include a plurality of layers which can vary within a proximal section flexible wall and within a distal section flexible wall. Examples of layered construction of the distal section flexible wall are discussed in greater detail below in reference to FIG. 6B. Examples of layered construction of the proximal section flexible wall are discussed in greater detail in reference to FIG. 7B.

FIGS. 5A-5C are simplified diagrams of an axial support structure 500 of a flexible elongate device, such as flexible elongate device 400, according to some embodiments. According to some embodiments consistent with FIGS. 1-4C, axial support structure 500 may correspond to axial support structure 424. Consistent with such embodiments, axial support structure 500 may be disposed within a flexible body, such as flexible body 410. However, it is to be understood that axial support structure 500 may be used in other contexts, including as a standalone device and/or in conjunction with components other than those described herein.

According to the embodiments shown in FIGS. 5A-5C, axial support structure 500 is formed as a spine composed of a plurality of hoops 511-519. Hoops 511-519 are coupled to each other by flexible struts 521-529. A channel 530 extends centrally through axial support structure 500 and provides a conduit for a main lumen, such as main lumen 411. Grooves 540 extend circumferentially through axial support structure 500 and provide conduits for one or more lumens, such as lumens 412 and 419. In some examples, grooves 540 may be formed on the inner surface of hoops 511-519, the outer surface of hoops 511-519, and/or any combination thereof. In some examples, grooves 540 may be fully enclosed by hoops 511-519, forming tunnels. The size and position of grooves 540 is discussed in greater detail below with reference to FIGS. 6A-6B.

As depicted in FIGS. 5A-5B, channel 530 and grooves 540 are at least partially enclosed by hoops 511-519, which serve to reinforce the tool delivery and/or control element lumens. According to some embodiments, reinforcing the lumen walls may protect against pull-through, which occurs when control elements (and/or other devices extending through the lumens) chafe against and eventually pull through the lumen walls.

According to some embodiments, axial support structure 500 may be formed monolithically. For example, hoops 511-519 and flexible struts 521-529 may be defined by cutting away material from a monolithic tube. This tends to reduce manufacturing and/or operational complexity relative to other types of support structures that include multiple different components. Gaps 531-539 are formed in the cut away regions of axial support structure 500. As depicted in FIG. 5A, gaps 531-539 have a capital 'I' shape with reliefs 560 and a convex middle section 561. Reliefs 560 define the length and width of flexible struts 521-529, which in turn impacts the flexibility of axial support structure 500, as longer, narrower struts are generally more flexible than shorter, wider struts. Convex middle section 561 defines the range of motion of flexible struts 521-529 by setting the amount by which flexible struts 521-529 can flex before adjacent hoops 511-519 touch.

Adjacent pairs of hoops 511-519 are rotated by 90 degrees with respect to each other. Consequently, adjacent pairs of flexible struts 521-529 flex along perpendicular axes. This arrangement provides at least two degrees of freedom to axial support structure 500. In this manner, axial support structure 500 may be flexed in any direction in response to applied actuation forces. In alternative embodiments, adjacent pairs of flexible struts 521-529 may be rotated by angles other than 90 degrees (e.g., 60 degrees and/or 45 degrees) to support different bending responses to applied actuation forces.

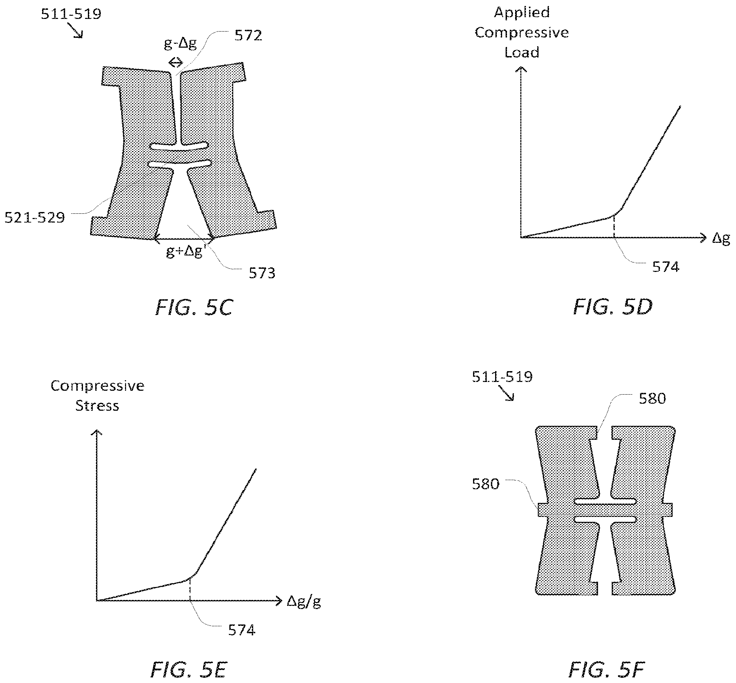

The steerability and/or range of motion of axial support structure 500 is determined by various parameters of axial support structure 500, including a hoop length 570, a gap length 571, and a number of hoops 511-519. During operation, one or more of flexible struts 521-529 bend due to actuation forces applied to axial support structure 500. FIG. 5C illustrates one of flexible struts 521-529 in a bent state. In response to bending of the flexible strut, gap length 571 reduces from a nominal length of g to a shorter length of g-Δg on an inner bend side 572 and wider from the nominal length of g to a longer g+Δg' on an outer bend side 573. In some embodiments, due to variations in material properties, the location of flexible struts 521-529, and/or the applied force causing bending, a change in gap length Δg of inner bend side 572 may not be equal to the change in gap length Δg' of outer bend side 573 such that when gap length 571 of inner bend side 572 may become g-Δg while gap length 571 of outer bend side 573 may become g+Δg'.

One figure of merit of axial support structure 500 is the ratio of Δg to g during operation. In some examples, the ability to accommodate a high gap ratio Δg/g facilitates steerability because a large bend may be produced at each set of struts. However, there may be a minimum gap length g-Δg of inner bend side 572 that should be maintained in order to provide effective performance of axial support structure 500 during bending. Referring to FIGS. 5D and 5E, an effect of bending on axial support structure 500 can be depicted by a compressive strain that would be applied to a removable compliant fixture (not shown) placed within the gap on inner bend side 572. For example, if the compliant fixture is placed within the gap on inner bend side 572 and axial support structure 500 is placed in higher bending, the compliant fixture begins to compress as it experiences increased compressive load from the two adjacent hoops. The relationship between the change in gap size Δg and applied load is illustrated in FIG. 5D. As the change in gap size Δg begins to increase, the applied compressive load shows a gradual linear increase. At a threshold compression 574, axial support structure 500 is compressed to a condensed configuration that prevents axial support structure 500 from easily compressing further causing the applied compressive load to begin increasing at a more rapid rate. A similar behavior can be seen in FIG. 5E which shows a relationship between compressive stress as measured by gap ratio Δg/g and compressive strain, where compressive stress is related to the applied compressive load experienced the axial support structure 500. It thus can be desirable to operate within a range where the increase in compressive stress is small, by selecting a minimum gap length 571 of gap length g-Δg corresponding to threshold compression 574 or alternatively a maximum gap ratio 575 in FIG. 5E. Operating subject to one or both of these thresholds facilitates steerability while avoiding excessive compressive load. Depending on the materials used to construct a jacket layer (such as jacket layer 633) surrounding axial support structure 500, maximum gap ratio 575 can be set at 30 percent or within a range such as 20 to 40 percent . In alternative embodiments, different materials can provide for a different range of maximum gap ratio 575.

Increasing the number of hoops within the axial support structure 500 may also provide for a lower gap ratio and may provide for improved steerability. In one example, gap length 571 is decreased due to the larger number of hoops and the Δg is also decreased because each flexible strut 521-529 provides less bend for axial support structure 500 to reach a certain overall bend. By adjusting the number of hoops and gap length 571, the gap ratio Δg/g can be optimized. However, increasing the number of hoops may result in puckering, by which excess material gathers on the inner portion of the bend. In general, the degree of puckering is governed by the Poisson ratio of the material; a lower Poisson's ratio results in less puckering. Puckering may be problematic in some applications, as puckering may result in channel 530 being narrower and/or bumpier, and/or may increase wear on the flexible body. For example, puckering may place strain on a lumen wall layer and/or an adhesion layer of the flexible body, eventually causing the lumen wall layer to delaminate from other layers of the flexible body. In some embodiments, puckering may be minimized or avoided regardless of the number of hoops what the gap ratio is suitably limited. When the gap ratio is not suitably limited, puckering effects can be more apparent. Puckering will be even more excessive when the gap ratio is not suitably limited and the number of hoops is increased.

One way to maintain a low gap ratio of is to decrease hoop length 570 and/or increase the gap length 571 (e.g. long struts 521-529 and/or short hoops 511-519). In some examples, increasing the length of struts 521-529 and/or decreasing hoop length 570 may decrease the axial stiffness of axial support structure 500. Accordingly, hoop length 570 and gap length 571 may be selected to achieve a desired axial strength while mitigating puckering. In some embodiments, one or more protrusions 580 extending from each hoop 511-519, as shown in FIG. 5F, can act as a stopper preventing axial support structure 500 from further axial compression. In alternative embodiments (not shown), the one or more protrusions 580 may be positioned circumferentially closer to struts 521-529, i.e., along a circumference of hoops 511-519.

In some examples, the total length of axial support structure 500 may be 3.5 centimeters to 5.0 centimeters. For example, axial support structure 500 may include at least 30 hoops 511-519 (e.g., 34-37 hoops) to provide a satisfactory range of motion and a smooth channel 530. In some examples, the size of hoops 511-519 and/or struts 521-529 may vary along the length of axial support structure 500 to vary the flexibility of axial support structure 500 along its length. For example, axial support structure 500 may be stiffer at the proximal end (e.g., longer hoops, shorter and/or wider struts, and/or the like) and more flexible at the distal end (e.g., shorter hoops, longer and/or narrower struts, and/or the like) to effectuate a larger bending response to actuation forces near the distal tip of axial support structure 500.

FIGS. 6A-6B are simplified diagrams of a distal section 600 of a flexible elongate device, such as flexible elongate device 400, according to some embodiments. According to some embodiments consistent with FIGS. 1-5C, distal section 600 may correspond to distal section 404 of flexible elongate device 400. However, it is to be understood that distal section 600 may be used in contexts other than flexible elongate device 400, including as a standalone device and/or in conjunction with sections other than those of flexible elongate device 400.

Distal section 600 includes a flexible body 610 having a main lumen 611, four control element lumens 612 through which control elements 621 extend, and a sensor lumen 619 through which a localization sensor 626 extends. The distal section 600 can further include, an axial support structure 624. These features generally correspond to similarly labeled features of flexible elongate device 400, as described above. In some examples, axial support structure 624 may correspond to axial support structure 500.

As depicted in FIG. 6B, axial support structure 624 has a ring-like cross sectional shape that surrounds a central channel corresponding to main lumen 611. In some examples, grooves 625 may be formed on the surface of axial support structure 624 to accommodate lumens 612 and 619. Because lumens 612 and 619 are embedded within axial support structure 624, the presence of lumens 612 and 619 has little or no impact on the diameter of distal section 600 and/or main lumen 611. That is, the presence of lumens 612 and 619 neither increases the outer diameter of distal section 600 nor decreases the diameter of main lumen 611.

In some examples, grooves 625 may be formed on the inner and/or outer surface of axial support structure 624. Alternately or additionally, axial support structure may 624 may include tunnels, appendages (e.g., clips, tabs, etc.), and/or the like, to accommodate lumens 612 and 619. In some examples, the sizes of grooves 625 may generally match the size of corresponding lumens 612 and 619 and/or may provide sufficient clearance to allow instruments (e.g., control elements 621 and/or localization sensor 626) within the lumens to move freely in the axial direction while constraining their lateral motion. For example, grooves 625 may hold control elements 621 laterally in place to maintain steering alignment, prevent pull-through, and/or to prevent control elements 621 from drifting during operation, resulting in loss of steering control.

In some examples, the placement of localization sensor 626 around the circumference of axial support structure 624 may impact the accuracy of the corresponding localization sensor. For example, when axial support structure 624 includes struts, such as struts 521-529, the accuracy of the location sensor may depend on whether localization sensor 626 is aligned with the struts or is offset from the struts. Consequently, the placement of localization sensor 626 (and corresponding features that accommodate localization sensor 626, such as grooves 625 and/or sensor lumen 619) may be selected by determining the amount of offset from the struts that results in the greatest accuracy of the localization sensor.

Likewise, the placement of control elements 621 around the circumference of axial support structure 624 may impact the steerability of distal section 600. For example, when axial support structure 624 includes struts, such as struts 521-529, the steerability of distal section 600 may depend on whether control elements 621 are aligned with the struts or are offset from the struts. In some examples, when the size of control elements 621 and/or localization sensor 626 is similar to and/or greater than the thickness of the struts of axial support structure 624, control elements 621 may be offset from the struts. This may prevent grooves 625 from compromising the axial stiffness of axial support structure 624 by significantly reducing the thickness of the struts. Consequently, the placement of control elements 621 (and corresponding features that accommodate control elements 621, such as grooves 625 and/or control element lumens 612) may be selected by determining the amount of offset from the struts that results in the greatest steerability and/or satisfactory axial stiffness. When there is a conflict between the desired placement of localization sensor 626 and control elements 621, one or both of the conflicting lumens may be shifted to accommodate the other.

Flexible body 610 of distal section 600 can include a distal section flexible wall formed as a plurality of layers 630-639. A lumen wall layer 630 forms an inner lining around main lumen 611. In some examples, lumen wall layer 630 may be formed using a material with low friction and/or a high melting point, such as polytetrafluoroethylene (PTFE). A reinforcement layer 632 is disposed around control element lumens 612 and/or axial support structure 624. In some examples, reinforcement layer 632 may be formed from coiled and/or braided wires. The wires may be formed from various metal and/or polymer materials. In some examples, one or both ends of the braided reinforcement layer 632 may be surrounded by a a band to reduce fraying and/or to reduce damage to other layers of axial support structure 624 due to fraying of the braided reinforcement layer 632. In some examples, the band may include multiple loops of a single braid wire. In some examples, the band may be welded in place. In some examples, the band may include stainless steel.

A jacket layer 633 forms the outer coating of flexible body 610. In some examples, jacket layer 633 may be formed using a heat shrink tubing, vacuum-expanded tubing, and/or a material that melts when heated (e.g., a thermoplastic such as polyamide, polyether, silicone, and/or the like), an elastomer that may be stretched (e.g., ethylene propylene diene monomer rubber (EPDM), Vitron, and/or the like), and/or the like. Consistent with such embodiments, jacket layer 633 may fill in regions around lumens 611-619, reinforcement layer 632, and/or axial support structure 624 when heated.

In some examples, lumen wall layer 630 may have direct interfaces with axial support structure 624, reinforcement layer 632, jacket layer 633, and/or other layers of flexible body 610. To prevent delamination of lumen wall layer 630 at these interfaces, a tie layer 639 may be applied as a thin coating around lumen wall layer 630. In some examples, tie layer 634 may include a layer of polymer, such as polyether block amide (PEBA), polyurethane, and/or the like, that has better adhesion properties than the material of lumen wall layer 630 (e.g., PTFE).

In some embodiments, one or more of the layers 630-639 may be treated to increase adhesion to adjacent layers of the flexible body. For example, an outer surface of lumen wall layer 630 (and/or other PTFE layers) may be chemically etched. In some examples, one or more of the layers 630-639 may be mechanically roughened (e.g., sanded, grit blasted, and/or the like) to increase adhesion. Consistent with such embodiments, the optional tie layer 639 may be omitted.

Although FIGS. 6A-6B illustrates one example of a sequence of layers 630-639 used to form flexible body 610, many different sequences of layers are possible. In some embodiments, grooves 625 may be positioned on the inner surface of axial support structure 624. Consistent with such embodiments, reinforcement layer 632 may be positioned on the inside of axial support structure 624, directly encapsulating lumen wall layer 630 and/or tie layer 639. In some embodiments, the sequence of layers may be selected to prevent material, such as the material of jacket layer 633, from entering gaps in axial support structure 624, such as gaps 531-539 of axial support structure 500. For example, reducing or eliminating the material in the gaps may increase the flexibility of axial support structure 624, reduce puckering, and/or the like.

One way to provide air gaps between hoops of axial support structure 624 is to encapsulate axial support structure 624 between an inner and an outer liner, such as PTFE. The inner and outer liners may not melt during heat shrink processing. Accordingly, materials that do melt are prevented from entering the gaps of axial support structure 624 during the heat shrink processing. In some examples, the inner and outer liners may further provide a low-friction surface to allow axial support structure 624 to slide axially relative to the other layers during actuation, which may improve steerability. Another way to provide air gaps between hoops of axial support structure 624 is to form the sequence of layers 630-639 using process steps that do not involve melting the layers. For examples, jacket layer 633 may include a vacuum-expanded jacket, a jacket that is loose-fitting around axial support structure 624, a jacket that is adhesively bonded at one or more ends of the flexible elongate device, and/or a cross-linked jacket that shrinks, but does not melt, during heat shrink processing.

In some embodiments, an illustrative sequence of layers that may be used to provide air gaps between hoops of axial support structure 624 (starting from the inner layer) include: (1) a lumen wall layer, e.g., PTFE; (2) an adhesion layer, e.g., PEBA; (3) a reinforcement layer, e.g., metal braid; (4) an inner air gap liner, e.g., PTFE; (5) an axial support structure 624 with air gaps; (6) an outer air gap liner, e.g., PTFE; and (7) a jacket layer, e.g., thermoplastic. Because axial support structure 624 is encapsulated by liner layers that do not melt when the other layers are heated(e.g., inner and outer PTFE layers (4) and (6)), the jacket layer material does not enter the air gaps of axial support structure 624 during the heat shrink process.

In some embodiments, another illustrative sequence of layers that may be used to provide air gaps between hoops of axial support structure 624 (starting from the inner layer) include: (1) a lumen wall layer, e.g., PTFE; (2) an adhesion layer, e.g., PEBA; (3) an axial support structure 624 with air gaps; (4) a reinforcement layer, e.g., metal braid; (5) a partial jacket layer, e.g., thermoplastic filling in a portion of axial support structure 624; and (6) an outer elastomeric jacket layer encapsulating (1)-(5) allowing partially exposed air pockets within axial support structure 624.

FIGS. 7A-7B are simplified diagrams of a portion of a proximal section 700 of a flexible elongate device, such as flexible elongate device 400, according to some embodiments. According to some embodiments consistent with FIGS. 1-6B, proximal section 700 may correspond to proximal section 402 of flexible elongate device 400 and include a flexible body 710 corresponding to flexible body 410. However, it is to be understood that proximal section 700 may be used in contexts other than flexible elongate device 400, including as a standalone device and/or in conjunction with sections other than those of flexible elongate device 400.

The flexible body 710 includes a main lumen 711, control element lumens 712, and sensor lumen 719 each extending from a proximal end to a distal end of the flexible body 710. As depicted in FIGS. 7A-7B, the proximal section 700 can include four control element lumens 712, a sensor lumen 719, control elements 721, and conduits 723, These features generally correspond to similarly labeled features of flexible elongate device 400, as described above.

Flexible body 710 of proximal section 700 can include a proximal section flexible wall formed as a plurality of layers 730-739. A lumen wall layer 730 forms an inner lining around main lumen 711. In some examples, lumen wall layer 730 may be formed using a material with low friction and/or a high melting point, such as PTFE. A lumen reinforcement layer 731 is formed around lumen wall layer 730. In some examples, lumen reinforcement layer 731 may be formed from braided and/or coiled wires. The wires may be formed from various metal and/or polymer materials. An outer reinforcement layer 732 is disposed around control element lumens 712. Like lumen reinforcement layer 731, outer reinforcement layer 732 may be formed from braided and/or coiled wires. A jacket layer 733 forms the outer coating of flexible body 710. In some examples, jacket layer 733 may be formed using a heat shrink tubing and/or a material that melts under when heated (e.g., a thermoplastic such as polyamide, polyether, silicone, and/or the like), an elastomer that may be stretched (e.g., EPDM, Vitron, and/or the like), and/or the like. Consistent with such embodiments, jacket layer 733 may fill in regions around lumens 712 and/or 719 and/or layers 731 and/or 732 when heated. In some examples consistent with FIGS. 6A-6B, one or more of layers 730-739 may be contiguous with and/or formed using the same materials as corresponding layers 630-639. For example, lumen wall layer 730 and/or jacket layer 733 may be contiguous with and/or formed using the same materials as lumen wall layer 630 and/or jacket layer 633, respectively.

In some examples, lumen wall layer 730 may directly interface with lumen reinforcement layer 731, jacket layer 733, and/or other layers of flexible body 710. To prevent delamination of lumen wall layer 730 at these interfaces, an optional tie layer 739 may be applied as a thin coating around lumen wall layer 730. In some examples, tie layer 739 may include a layer of polymer, such as polyether block amide (PEBA), that has better adhesion properties than the material of lumen wall layer 730 (e.g., PTFE).

Lumen wall layer 730 and lumen reinforcement layer 731 generally have the same cross-sectional shape as main lumen 711. In the example shown in FIG. 7B, the cross-sectional shape corresponds to a rounded square rotated by approximately 30 degrees relative to a rounded square formed by outer reinforcement layer 732, as described above with reference to FIG. 4B. This arrangement makes efficient use of the cross-sectional area of proximal section 700. In particular, main lumen 711 occupies a large cross-sectional area in the center of proximal section 700, while lumens 712 and 719 are efficiently arranged into triangular corner regions formed by rotating main lumen 711 relative to outer reinforcement layer 732. Moreover, in this arrangement, each of lumens 711, 712, and 719 is encapsulated within at least one reinforcement layer (e.g., lumen reinforcement layer 731 and/or outer reinforcement layer 732). In addition, main lumen 711 is separated from each of lumens 712 and 719 by at least one reinforcement layer. Accordingly, the arrangement of flexible layers shown in FIG. 7B enhances the structural integrity of proximal section 700 and main lumen 711 while making efficient use of the available cross-sectional area.

FIGS. 8A-8B are simplified diagrams of a stopper 800 of a flexible elongate device, such as flexible elongate device 400, according to some embodiments. According to some embodiments consistent with FIGS. 1-7B, stopper 800 may correspond to stopper 425 of flexible elongate device 400. However, it is to be understood that stopper 800 of may be used in contexts other than flexible elongate device 400, including as a standalone device and/or in conjunction with components other than those of flexible elongate device 400.

Stopper 800 prevents components, such as conduits 423 and/or axial support structure 424, from shifting axially into adjoining sections during operation. In some examples, stopper 800 may be formed as a solid block with a channel 810 and a plurality of grooves 811. In some examples consistent with FIGS. 5A-5C, the size of channel 810 may match the size of channel 530 of axial support structure 500. In some examples, when conduits 423 include conduits, the size of grooves 811 that abut the conduits may match and/or may be smaller than the size of the conduits such that the conduits are prevented from sliding through grooves 811. At the same time, grooves 811 may be sufficiently large to allow instruments such as control elements 421 and/or localization sensor 426 to pass through.

FIG. 9 is a simplified diagram of a distal mount 900 of a flexible elongate device, such as flexible elongate device 400, according to some embodiments. According to some embodiments consistent with FIGS. 1-8B, distal mount 900 may correspond to distal mount 422 of flexible elongate device 400. However, it is to be understood that distal mount 900 may be used in contexts other than flexible elongate device 400, including as a standalone device and/or in conjunction with components other than those of flexible elongate device 400.

Distal mount 900 includes a control ring 910 coupled to a tip ring 920. As depicted in FIG. 9, control ring 910 is located adjacent to an axial support structure 930 corresponding to axial support structure 424 of flexible elongate device 400. Consistent with such embodiments, control ring 910 may have cross-sectional dimensions matching axial support structure 930. In some examples, tip ring 920 and control ring 910 may have matching tapers such that tip ring 920 slides on to a distal portion of control ring 910. In some examples, a liner 922 can be positioned between control ring 910 and tip ring 920 and tip ring 920 may be soldered, laser welded, adhesively bonded, and/or the like to control ring 910 at a location 924 at the distal tip of the distal mount 900. In this example, liner 922 would provide a seal between the control ring 910 and tip ring 920. In order to provide an adequate seal, the control ring 910 can flare around the tip ring 920 creating a flared distal mount. In some examples tip ring 920 and control ring 910 may be composed of a thermoplastic material, stainless steel, gold plated 304 stainless steel, and/or the like.

Control elements 940, which may correspond to control elements 421 of flexible elongate device 400, are fixedly attached to control ring 910. In some examples, control elements 940 may be soldered, laser welded, adhesively bonded, and/or the like to control ring 910 at bonding points 941. A localization sensor 950, which may correspond to localization sensor 426 of flexible elongate device 400, is also fixedly attached to control ring 910. In some examples, localization sensor 950 may be adhesively bonded to control ring 910. In alternative examples, localization sensor 950 may be held in place by a flexible body in which distal mount 900 is disposed, such as flexible body 410. In some embodiments, control elements 940 and/or localization sensor 950 may be pinned between control ring 910 and tip ring 920 where tip ring 920 tapers around control ring 910.

FIG. 10 is a simplified diagram of a flexible body 1000 with a keyed lumen according to some embodiments. According to some embodiments consistent with FIGS. 1-9, flexible body may correspond to flexible body 410 of flexible elongate device 400. However, it is to be understood that flexible body 1000 of may be used in contexts other than flexible elongate device 400, including as a standalone device and/or in conjunction with components other than those of flexible elongate device 400. Flexible body 1000 includes a plurality of lumens 1011-1119, which generally correspond to lumens 411-419 of flexible body 410. Flexible body 1000 may further include one or more reinforcement layers 1022 and 1024, which generally correspond to layers 632, 731, and/or 732.

As depicted in FIG. 10, main lumen 711 of flexible body 1000 is keyed. That is, main lumen 711 has a non-circular cross-sectional shape that prevents or constrains the rotation of an instrument with a matching non-circular cross-sectional shape when inserted through main lumen 711. In general, the symmetry of the cross-sectional shape determines how many keyed orientations are supported. As depicted in FIG. 10, main lumen 711 has a cross-sectional shape that is symmetric about a single axis (e.g., a vertical axis through the center of main lumen 711) that results in a single possible orientation for inserting a similarly shaped and slightly smaller device within main lumen 711. And although FIG. 10 shows one possible shape with symmetry about a single axis, many other shapes are possible with symmetry about a single axis, such as an isosceles triangle, an isosceles triangle with rounded corners, and/or the like. Other shapes with symmetry about more axes, and thus more possible keyed orientations are also possible. For example, a cross-sectional shape with symmetry about four axes (e.g., a square and/or a square with rounded corners, and/or a square with beveled corners) supports four different keyed orientations, a cross-sectional shape with symmetry about two axes (e.g., an ellipse, a diamond, a diamond with rounded corners, a rectangle, and/or a rectangle with rounded corners) supports two different keyed orientations, a cross-sectional shape with symmetry about three axes (e.g., an equilateral triangle), and/or the like. More generally, the cross sectional shape of main lumen 711 may have symmetry about n-axes to support n keyed orientations.

One illustrative application of a keyed lumen, such as main lumen 711, is to maintain a desired roll orientation of an endoscope to provide a properly oriented camera image to the user. Consistent with such embodiments, a visual orientation indicator may be placed on the inner lining of main lumen 711. For example, the visual orientation indicator may include a colored stripe on one side of the inner lining of main lumen 711. When the endoscope is inserted into main lumen 711, the visual orientation indicator appears in the camera image generated by the endoscope. In some examples, machine vision processing may be used to automatically detect the visual orientation indicator in the camera image and rotate the camera image to a desired orientation for display.

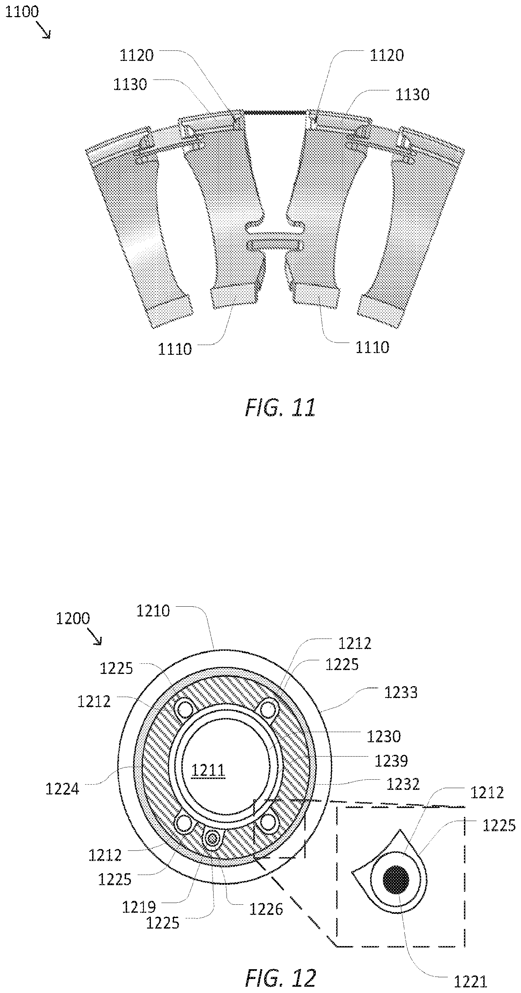

FIG. 11 is a simplified diagram of an axial support structure 1100 of a flexible elongate device, such as flexible elongate device 400, according to some embodiments. According to some embodiments consistent with FIGS. 1-4C, axial support structure 1100 may correspond to axial support structure 424. Consistent with such embodiments, axial support structure 1100 may be disposed within a flexible body, such as flexible body 410. However, it is to be understood that axial support structure 1100 may be used in other contexts, including as a standalone device and/or in conjunction with components other than those described herein.

According to the embodiments shown in FIG. 11, axial support structure 1100 is formed as a spine composed of a plurality of hoops 1110. Hoops 1110 are coupled to each other by flexible struts consistent with those depicted with respect to hoops 511-519. A channel extends centrally through axial support structure 1100 and provides a conduit for a main lumen, such as main lumen 411. Grooves 1120 extend circumferentially through axial support structure 1100 and provide conduits for one or more lumens, such as lumens 412 and 419. In some examples, grooves 1120 may be formed on the inner surface of hoops 1110, the outer surface of hoops 1110, and/or any combination thereof. In some examples, grooves 1120 may be fully enclosed by hoops 1110, forming tunnels.

As depicted in FIG. 11, grooves 1120 are at least partially enclosed by hoops 1110, which serve to reinforce the tool delivery and/or control element lumens. According to some embodiments, reinforcing the lumen walls may protect against pull-through, which occurs when control elements (and/or other devices extending through the lumens) chafe against and eventually pull through the lumen walls.

As further depicted in FIG. 11, grooves 1120 include a cutout 1130 at each of a corresponding hoop 1110 in order to reduce pinching and/or damage to control elements, a localization sensor, and/or the like deployed within grooves 1120 when axial support structure 1100 is bent, flexed, and/or the like. In some examples, cutouts 1130 may include one or more of a circular shape, a beveled edge, and/or the like.

FIG. 12 is a simplified diagram of a distal section 1200 of a flexible elongate device, such as flexible elongate device 400, according to some embodiments. According to some embodiments consistent with FIGS. 1-5C, distal section 1200 may correspond to distal section 404 of flexible elongate device 400. However, it is to be understood that distal section 1200 may be used in contexts other than flexible elongate device 400, including as a standalone device and/or in conjunction with sections other than those of flexible elongate device 400.

Distal section 1200 includes a flexible body 1210 having a main lumen 1211, four control element lumens 1212 through which control elements 1221 extend, and a sensor lumen 1219 through which a localization sensor 1226 extends. The distal section 1200 can further include, an axial support structure 1224. These features generally correspond to similarly labeled features of flexible elongate device 400, as described above. In some examples, axial support structure 1224 may correspond to axial support structure 500.

As depicted in FIG. 12, axial support structure 1224 has a ring-like cross sectional shape that surrounds a central channel corresponding to main lumen 1211. In some examples, grooves 1225 may be formed on an inner surface of axial support structure 1224 to accommodate lumens 1212 and 1219. Because lumens 1212 and 1219 are embedded within axial support structure 1224, the presence of lumens 1212 and 1219 has little or no impact on the diameter of distal section 1200 and/or main lumen 1211. That is, the presence of lumens 1212 and 1219 neither increases the outer diameter of distal section 1200 nor decreases the diameter of main lumen 1211.

In some examples, the sizes of grooves 1225 may generally match the size of corresponding lumens 1212 and 1219 and/or may provide sufficient clearance to allow instruments (e.g., control elements 1221 and/or localization sensor 1226) within the lumens to move freely in the axial direction while constraining their lateral motion. For example, grooves 1225 may hold control elements 1221 laterally in place to maintain steering alignment, prevent pull-through, and/or to prevent control elements 1221 from drifting during operation, resulting in loss of steering control. In some examples, placement of grooves 1225 on the inner surface of axial support structure 1224 may reduce tension in control elements 1221 and/or localization sensor 1226, such as tension used to steer distal section 1200 and/or tension due to bending and/or flexing of axial support structure 1224.

In some examples, the placement of localization sensor 1226 around the inner circumference of axial support structure 1224 may impact the accuracy of the corresponding localization sensor. For example, when axial support structure 1224 includes struts, such as struts 521-529, the accuracy of the location sensor may depend on whether localization sensor 12212 is aligned with the struts or is offset from the struts. Consequently, the placement of localization sensor 1226 (and corresponding features that accommodate localization sensor 1226, such as grooves 1225 and/or sensor lumen 1219) may be selected by determining the amount of offset from the struts that results in the greatest accuracy of the localization sensor.

Likewise, the placement of control elements 1221 around the inner circumference of axial support structure 1224 may impact the steerability of distal section 1200. For example, when axial support structure 1224 includes struts, such as struts 521-529, the steerability of distal section 1200 may depend on whether control elements 1221 are aligned with the struts or are offset from the struts. In some examples, when the size of control elements 1221 and/or localization sensor 1226 is similar to and/or greater than the thickness of the struts of axial support structure 1224, control elements 1221 may be offset from the struts. This may prevent grooves 1225 from compromising the axial stiffness of axial support structure 1224 by significantly reducing the thickness of the struts. Consequently, the placement of control elements 1221 (and corresponding features that accommodate control elements 1221, such as grooves 1225 and/or control element lumens 1212) may be selected by determining the amount of offset from the struts that results in the greatest steerability and/or satisfactory axial stiffness. When there is a conflict between the desired placement of localization sensor 1226 and control elements 1221, one or both of the conflicting lumens may be shifted to accommodate the other.

Flexible body 1210 of distal section 1200 can include a distal section flexible wall formed as a plurality of layers 1230-1239. A lumen wall layer 1230 forms an inner lining around main lumen 1211. In some examples, lumen wall layer 1230 may be formed using a material with low friction and/or a high melting point, such as polytetrafluoroethylene (PTFE). A reinforcement layer 1232 is disposed around control element lumens 1212 and/or axial support structure 1224. In some examples, reinforcement layer 1232 may be formed from coiled and/or braided wires. The wires may be formed from various metal and/or polymer materials. A jacket layer 1233 forms the outer coating of flexible body 1210. In some examples, jacket layer 1233 may be formed using a heat shrink tubing, vacuum-expanded tubing, and/or a material that melts when heated (e.g., a thermoplastic such as polyamide, polyether, silicone, and/or the like), an elastomer that may be stretched (e.g., ethylene propylene diene monomer rubber (EPDM), Vitron, and/or the like), and/or the like. Consistent with such embodiments, jacket layer 1233 may fill in regions around lumens 1211-1219, reinforcement layer 1232, and/or axial support structure 1224 when heated.

In some examples, lumen wall layer 1230 may have direct interfaces with axial support structure 1224, reinforcement layer 1232, jacket layer 1233, and/or other layers of flexible body 1210. To prevent delamination of lumen wall layer 1230 at these interfaces, a tie layer 1239 may be applied as a thin coating around lumen wall layer 1230. In some examples, tie layer 1234 may include a layer of polymer, such as polyether block amide (PEBA), polyurethane, and/or the like, that has better adhesion properties than the material of lumen wall layer 1230 (e.g., PTFE).

In some embodiments, one or more of the layers 1230-1239 may be treated to increase adhesion to adjacent layers of the flexible body. For example, an outer surface of lumen wall layer 1230 (and/or other PTFE layers) may be chemically etched. In some examples, one or more of the layers 1230-1239 may be mechanically roughened (e.g., sanded, grit blasted, and/or the like) to increase adhesion. Consistent with such embodiments, the optional tie layer 1239 may be omitted.

One or more elements in embodiments of this disclosure may be implemented in software to execute on a processor of a computer system such as control processing system. When implemented in software, the elements of the embodiments of the invention are essentially the code segments to perform the necessary tasks. The program or code segments can be stored in a processor readable storage medium or device that may have been downloaded by way of a computer data signal embodied in a carrier wave over a transmission medium or a communication link. The processor readable storage device may include any medium that can store information including an optical medium, semiconductor medium, and magnetic medium. Processor readable storage device examples include an electronic circuit; a semiconductor device, a semiconductor memory device, a read only memory (ROM), a flash memory, an erasable programmable read only memory (EPROM); a floppy diskette, a CD-ROM, an optical disk, a hard disk, or other storage device. The code segments may be downloaded via computer networks such as the Internet, Intranet, etc. Any of a wide variety of centralized or distributed data processing architectures may be employed. Programmed instructions may be implemented as a number of separate programs or subroutines, or they may be integrated into a number of other aspects of the systems described herein. In one embodiment, the control system supports wireless communication protocols such as Bluetooth, IrDA, HomeRF, IEEE 802.11, DECT, and Wireless Telemetry.

Medical tools that may be delivered through the flexible elongate devices or catheters disclosed herein may include, for example, image capture probes, biopsy instruments, laser ablation fibers, and/or other surgical, diagnostic, or therapeutic tools. Medical tools may include end effectors having a single working member such as a scalpel, a blunt blade, an optical fiber, an electrode, and/or the like. Other end effectors may include, for example, forceps, graspers, scissors, clip appliers, and/or the like. Other end effectors may further include electrically activated end effectors such as electrosurgical electrodes, transducers, sensors, and/or the like. Medical tools may include image capture probes that include a stereoscopic or monoscopic camera for capturing images (including video images). Medical tools may additionally house cables, linkages, or other actuation controls (not shown) that extend between its proximal and distal ends to controllably bend the distal end of medical instrument 226. Steerable instruments are described in detail in U.S. Patent No. 7,316,681 (filed on Oct. 4, 2005) (disclosing "Articulated Surgical Instrument for Performing Minimally Invasive Surgery with Enhanced Dexterity and Sensitivity") and U.S. Patent No. 9,259,274 (filed Sept. 30, 2008) (disclosing "Passive Preload and Capstan Drive for Surgical Instruments"), which are incorporated by reference herein in their entireties.

The systems described herein may be suited for navigation and treatment of anatomic tissues, via natural or surgically created connected passageways, in any of a variety of anatomic systems, including the lung, colon, the intestines, the kidneys and kidney calices, the brain, the heart, the circulatory system including vasculature, and/or the like.

Note that the processes and displays presented may not inherently be related to any particular computer or other apparatus. Various general-purpose systems may be used with programs in accordance with the teachings herein, or it may prove convenient to construct a more specialized apparatus to perform the operations described. The required structure for a variety of these systems will appear as elements in the claims. In addition, the embodiments of the invention are not described with reference to any particular programming language. It will be appreciated that a variety of programming languages may be used to implement the teachings of the invention as described herein.

While certain exemplary embodiments of the invention have been described and shown in the accompanying drawings, it is to be understood that such embodiments are merely illustrative of and not restrictive on the broad invention, and that the embodiments of the invention not be limited to the specific constructions and arrangements shown and described, since various other modifications may occur to those ordinarily skilled in the art.

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A flexible elongate device comprising:
   a flexible body and an axial support structure, wherein the axial support structure includes at least one groove extending along a length of the axial support structure; and
   a plurality of control elements for actuating the flexible elongate device, wherein each of the plurality of control elements extends through one of the at least one groove of the axial support structure.
2. The flexible elongate device of clause 1, wherein the at least one groove includes a plurality of grooves spaced circumferentially around the axial support structure.
3. The flexible elongate device of clause 1, wherein the flexible body includes a plurality of control element lumens that extend through the at least one groove of the axial support structure, the plurality of control elements being disposed in the plurality of control element lumens.
4. The flexible elongate device of clause 1, wherein the flexible body includes a main lumen that extends centrally through the flexible body, the main lumen providing a channel for a medical tool.
5. The flexible elongate device of clause 4, wherein the main lumen is keyed.
6. The flexible elongate device of any one of clauses 1-5, wherein a cross-sectional shape of the main lumen has symmetry about n-axes, where n is selected from a group consisting of 1, 2, 3, and 4.
7. The flexible elongate device of any one of clauses 1-5, wherein an inner lining of the main lumen includes a visual orientation indicator to indicate an orientation in an endoscope camera image.
8. The flexible elongate device of any one of clauses 1-5, wherein the flexible body comprises a sequence of layers including a lumen wall layer and a jacket layer.
9. The flexible elongate device of clause 8, wherein the lumen wall layer comprises polytetrafluoroethylene (PTFE), and the jacket layer comprises a thermoplastic.
10. The flexible elongate device of clause 8, wherein the sequence of layers further comprises an inner air gap liner and an outer air gap liner forming air gaps within the axial support structure that are not filled in by the jacket layer.
11. The flexible elongate device of clause 10, wherein the inner air gap liner and the outer air gap liner are composed of PTFE.
12. The flexible elongate device of clause 8, wherein the sequence of layers further comprises at least one reinforcement layer.
13. The flexible elongate device of clause 12, wherein the at least one reinforcement layer comprises a metal braid.
14. The flexible elongate device of clause 8, wherein the sequence of layers further comprises an adhesion layer between the lumen wall layer and other layers in the sequence of layers.
15. The flexible elongate device of clause 14, wherein the adhesion layer is composed of polyether block amide (PEBA).
16. The flexible elongate device of any one of clauses 1-5, wherein:
   the flexible elongate device includes a proximal section and a distal section, and wherein
   the axial support structure is disposed within the distal section.
17. The flexible elongate device of clause 16 further comprising a conduit within the proximal section of the flexible elongate device, the conduit transferring an applied force to one of the control elements from a proximal end of the proximal section to a distal end of the proximal section.
18. The flexible elongate device of clause 17, wherein the conduit is disposed coaxially around the one of the control elements.
19. The flexible elongate device of clause 17, wherein the flexible elongate device further includes a transition section, and a stopper within the transition section, wherein the stopper prevents the conduit from moving distally during operation.
20. The flexible elongate device of any one of clauses 1-5, further comprising a distal mount at a distal end of the axial support structure, and wherein each of the control elements is fixedly attached to the distal mount.
21. The flexible elongate device of any one of clauses 1-5, further comprising a localization sensor, the localization sensor extending along the length of the axial support structure through at least one of the plurality of grooves of the axial support structure.
22. The flexible elongate device of any one of clauses 1-5, wherein the axial support structure includes a set of hoops with each adjacent pair of hoops being coupled by a corresponding pair of struts.
23. The flexible elongate device of clause 22, wherein gaps between the adjacent pairs of hoops have an 'I' shape with reliefs that define a length of the struts and a convex middle section that defines a range of motion of the axial support structure.
24. The flexible elongate device of clause 22, wherein a gap length between the hoops is selected such that a change in the gap length when the axial support structure is bent relative to the gap length in an unbent state remains below a predetermined threshold.
25. The flexible elongate device of clause 24, wherein the predetermined threshold corresponds to a ratio of the change in gap length to the gap length of 0.3.
26. A method of controlling a flexible elongate device having a flexible body and an axial support structure, the method comprising:
   actuating a plurality of control elements, each of the control elements extending through a corresponding one of at least one groove in the axial support structure.
27. The method of clause 26, further comprising deploying a medical tool through a main lumen that extends centrally through the flexible body.
28. The method of clause 27, wherein the main lumen is keyed to prevent rotation of the medical tool within the main lumen.
29. The method of clause 28, wherein a cross-sectional shape of the main lumen has symmetry about n-axes, where n is selected from a group consisting of 1, 2, 3, and 4.
30. The method of any one of clauses 27-29, further comprising:
   detecting a visual orientation indicator on an inner lining of the main lumen; and
   adjusting a display of an orientation of an endoscope camera image based on the detected visual orientation indicator.
31. The method of any one of clauses 26-29, wherein the flexible body includes a plurality of control element lumens extending through the at least one groove of the axial support structure, the plurality of control elements being disposed in the plurality of control element lumens.
32. The method of any one of clauses 26-29, further comprising steering a distal end of the flexible elongate device by applying unequal forces to each of the plurality of control elements.
33. The method of any one of clauses 26-29, wherein the flexible body comprises a sequence of layers including a lumen wall layer and a jacket layer.
34. The method of clause 33, wherein the lumen wall layer comprises polytetrafluoroethylene (PTFE), and the jacket layer comprises a thermoplastic.
35. The method of clause 33, wherein the sequence of layers further comprises an inner air gap liner and an outer air gap liner forming air gaps within the axial support structure that are not filled in by the jacket layer.
36. The method of clause 35, wherein the inner air gap liner and the outer air gap liner are composed of PTFE.
37. The method of clause 33, wherein the sequence of layers further comprises at least one reinforcement layer.
38. The method of clause 37, wherein the at least one reinforcement layer comprises a metal braid.
39. The method of clause 33, wherein the sequence of layers further comprises an adhesion layer between the lumen wall layer and other layers in the sequence of layers.
40. The method of clause 39, wherein the adhesion layer is composed of polyether block amide (PEBA).
41. The method of any one of clauses 26-29, wherein:
   the flexible elongate device includes a proximal section and a distal section, and wherein
   the axial support structure is disposed within the distal section.
42. The method of clause 41 further comprising a conduit within the proximal section of the flexible elongate device, the conduit transferring an applied force to at least one of the control elements from a proximal end of the proximal section to a distal end of the proximal section.
43. The method of clause 42, wherein the conduit is disposed coaxially around the at least one of the control elements.
44. The method of clause 42, wherein the flexible elongate device further includes a transition section, and a stopper within the transition section, wherein the stopper prevents the conduit from moving distally during operation.
45. The method of any one of clauses 26-29, further comprising a distal mount at a distal end of the axial support structure, and wherein each of the control elements is fixedly attached to the distal mount.
46. The method of any one of clauses 26-29, further comprising a localization sensor, the localization sensor extending along a length of the axial support structure through a second of the at least one groove of the axial support structure.
47. The method of any one of clauses 26-29, wherein the axial support structure includes a set of hoops with each adjacent pair of hoops being coupled by a corresponding pair of struts.
48. The method of clause 47, wherein gaps between the adjacent pairs of hoops have an 'I' shape with reliefs that define a length of the struts and a convex middle section that defines a range of motion of the axial support structure.
49. The method of clause 47, wherein a gap length between the hoops is selected such that a change in the gap length when the axial support structure is bent relative to the gap length in an unbent state remains below a predetermined threshold.
50. The method of clause 49, wherein the predetermined threshold corresponds to a ratio of the change in gap length to the gap length of 0.3.
51. A steerable catheter actuated by one or more control elements, the steerable catheter comprising:
   a proximal section including one or more conduits to transfer actuation forces applied to the one or more control elements from distal end to a proximal end of the proximal section;
   a transition section including a stopper to prevent the one or more conduits from moving axially along the steerable catheter; and
   a distal section including an axial support structure to support the distal section against axial loads generated by the one or more control elements.
52. The steerable catheter of clause 51, wherein the one or more control elements extend through one or more grooves in the axial support structure.
53. The steerable catheter of clause 52, wherein the one or more grooves comprise a plurality of grooves spaced circumferentially around the axial support structure.
54. The steerable catheter of clause 51, wherein the distal section includes a plurality of control element lumens that extend through one or more grooves of the axial support structure, the one or more control elements being disposed in the plurality of control element lumens.
55. The steerable catheter of any one of clauses 51-54, wherein the distal section includes a main lumen that extends centrally through the distal section, the main lumen providing a channel for a medical tool.
56. The steerable catheter of clause 55, wherein the main lumen is keyed.
57. The steerable catheter of clause 55, wherein a cross-sectional shape of the main lumen has symmetry about n-axes, where n is selected from a group consisting of 1, 2, 3, and 4.
58. The steerable catheter of clause 55, wherein an inner lining of the main lumen includes a visual orientation indicator to indicate an orientation in an endoscope camera image.
59. The steerable catheter of any one of clauses 51-54, wherein the distal section comprises a sequence of layers including a lumen wall layer and a jacket layer.
60. The steerable catheter of clause 59, wherein the lumen wall layer comprises polytetrafluoroethylene (PTFE), and the jacket layer comprises a thermoplastic.
61. The steerable catheter of clause 59, wherein the sequence of layers further comprises an inner air gap liner and an outer air gap liner forming air gaps within the axial support structure that are not filled in by the jacket layer.
62. The steerable catheter of clause 61, wherein the inner air gap liner and the outer air gap liner are composed of PTFE.
63. The steerable catheter of clause 59, wherein the sequence of layers further comprises at least one reinforcement layer.
64. The steerable catheter of clause 63, wherein the at least one reinforcement layer comprises a metal braid.
65. The steerable catheter of clause 59, wherein the sequence of layers further comprises an adhesion layer between the lumen wall layer and other layers in the sequence of layers.
66. The steerable catheter of clause 65, wherein the adhesion layer is composed of polyether block amide (PEBA).
67. The steerable catheter of any one of clauses 51-54, further comprising a distal mount at a distal end of the axial support structure, and wherein each of the control elements is fixedly attached to the distal mount.
68. The steerable catheter of any one of clauses 51-54, further comprising a localization sensor, the localization sensor extending along a length of the steerable catheter.
69. The steerable catheter of any one of clauses 51-54, wherein the axial support structure includes a set of hoops with each adjacent pair of hoops being coupled by a corresponding pair of struts.
70. The steerable catheter of clause 69, wherein gaps between the adjacent pairs of hoops have an 'I' shape with reliefs that define a length of the struts and a convex middle section that defines a range of motion of the axial support structure.
71. The steerable catheter of clause 69, wherein a gap length between the hoops is selected such that a change in the gap length when the axial support structure is bent relative to the gap length in an unbent state remains below a predetermined threshold.
72. The steerable catheter of clause 71, wherein the predetermined threshold corresponds to a ratio of the change in gap length to the gap length of 0.3.

## Claims

1. A steerable catheter comprising:
a plurality of control elements;
a proximal section including a plurality of conduits to transfer actuation forces applied to one or more of the control elements of the plurality of control elements from a proximal end to a distal end of the proximal section, wherein each control element of the plurality of control elements extends within a respective conduit of the plurality of conduits;
a transition section distal to the proximal section, the transition section including a stopper to prevent the plurality of conduits from moving axially along the steerable catheter, wherein the stopper comprises grooves for receiving the one or more control elements; and
a distal section distal to the transition section, the transition section including an axial support structure to support the distal section against axial loads generated by the one or more control elements,
wherein the stopper prevents the plurality of conduits from extending into the distal section and allows the plurality of control elements to extend into the distal section.

2. The steerable catheter of claim 1, wherein the grooves comprise a plurality of grooves spaced circumferentially around the stopper.

3. The steerable catheter of claim 1, wherein each groove receives a respective control element of the one or more control elements.

4. The steerable catheter of claim 1, wherein the grooves include a diameter that is smaller than a diameter of the conduits.

5. The steerable catheter of claim 4, wherein the diameter of the grooves is larger than a diameter of the one or more control elements.

6. The steerable catheter of claim 1, wherein the conduits are coupled to the stopper.

7. The steerable catheter of claim 1, wherein the stopper further prevents the axial support structure from moving axially along the steerable catheter.

8. The steerable catheter of claim 1, wherein the stopper further comprises a channel for a medical tool.

9. The steerable catheter of claim 1, wherein an outer diameter of the steerable catheter tapers at the transition section.

10. The steerable catheter of claim 1, further comprising:
a distal mount at a distal end of the axial support structure, wherein each of the control elements is fixedly attached to the distal mount.

11. The steerable catheter of claim 10, wherein each of the control elements is soldered to the distal mount.

12. The steerable catheter of claim 1, further comprising:
a localization sensor, the localization sensor extending along a length of the steerable catheter.

13. The steerable catheter of claim 1, wherein the one or more control elements extend through one or more grooves in the axial support structure.

14. The steerable catheter of claim 1, wherein the distal section comprises a sequence of layers including a lumen wall layer and a jacket layer.

15. The steerable catheter of claim 14, wherein the sequence of layers further comprises:
an inner air gap liner and an outer air gap liner forming air gaps within the axial support structure that are not filled in by the jacket layer;
a reinforcement layer; and
an adhesion layer between the lumen wall layer and other layers in the sequence of layers.
